# EUROPEAN PATENT APPLICATION

(11) **EP 4 800 108 A1**
(43) Date of publication of application: **02.09.2026**
(21) Application number: 24882366.8
(22) Date of filing: 22.10.2024
(51) Int. Cl.: C12N 15/09, A61K 35/17, A61P 35/00, C12N 5/10, C12N 5/0783, C12N 15/62, C12N 15/12, C12N 15/13

(54) **LONG-LIVED T CELLS, PHARMACEUTICAL COMPOSITION, AND METHOD OF USE**

(30) Priority: 23.10.2023 JP 2023181926
(71) Applicant: Keio University, Tokyo, 108-8345 (JP); Takara Bio Inc., Kusatsu-shi Shiga 525-0058 (JP); Aichi Prefecture, Nagoya-shi Aichi 460-0001 (JP)
(72) Inventor: KAGOYA, Yuki, Tokyo 160-8582 (JP); INOUE, Satoshi, Tokyo 160-8582 (JP); OKAMOTO, Sachiko, Kusatsu-shi, Shiga 525-0058 (JP); AMAISHI, Yasunori, Kusatsu-shi, Shiga 525-0058 (JP); NAGATA, Ryousuke, Kusatsu-shi, Shiga 525-0058 (JP)
(74) Representative: Klöckner, Christoph
(86) International application number: PCT/JP2024/037491
(87) International publication number: WO 2025/089255

(57) **Abstract**

Provided is a T cell that expresses a recombinant receptor, the T cell that satisfies the following (I) and (II):
(I) the T cell has reduced or eliminated expression and/or function of the PRDM1 gene; and
(II) the T cell has reduced or eliminated expression and/or function of the RCOR1 gene and/or has increased or activated expression of the c-Jun gene.

## Description

### Technical Field

The present invention relates to long-lived T cells, a pharmaceutical composition comprising the T cells, and a method of using the T cells.

### Background Art

Chimeric antigen receptor (CAR)-introduced T cell therapies are promising therapies capable of leading to curing of intractable cancers but have problems in that the infused T cells gradually age, and thus have the viability thereof impaired and lose cytotoxicity (i.e., are exhausted). Several genetic modification technologies for T cells for preventing these hypofunctions have been reported.

For example, Patent Literature 1 discloses imparting resistance to exhaustion to T cells through overexpression of the c-JUN gene which is an AP-1 transcription factor. However, Patent Literature 1 does not disclose attaining long-term viability of the T cells.

Non-Patent Literature 1 discloses imparting long-term viability to T cells through deletion of the PRDM1 gene. However, with deletion of the PRDM1 gene alone, the effector functions of the T cells are inhibited, and the T cells enter an exhausted state. In this literature, a method in which the Cas9 protein and a guide RNA are introduced through electroporation by utilizing the CRISPR/Cas9 technique is employed.

Non-Patent Literature 2 indicates that simultaneous knockout of the PRDM1 gene and the NR4A3 gene allows CAR-T cells to attain long-term viability and resistance to exhaustion. In this literature, electroporation is employed for genetic modification.

### Citation List

### Patent Literature

PTL 1: US11400117

### Non-Patent Literature

NPTL 1: Yoshikawa et al., Blood. 2022; 139 (14): 2156-2172
NPTL2: Jung In-Young et al., Sci Transl Med 2022; 14 (670): eabn7336

### Summary of Invention

### Technical Problem

Aging and exhaustion of T cells are regulated via different transcription networks, and there have been almost no developed methods for simultaneously improving these characteristics.

An object to be achieved by the present invention is to provide: T cells having long-term viability and resistance to exhaustion; a pharmaceutical composition comprising the T cells; and a method of using the T cells.

### Solution to Problem

The present invention includes embodiments described below.

### Item 1

AT cell that expresses a recombinant receptor, the T cell that satisfies the following (I) and (II):
(I) the T cell has reduced or eliminated expression and/or function of the PRDM1 gene; and
(II) the T cell has reduced or eliminated expression and/or function of the RCOR1 gene and/or has increased or activated expression of the c-Jun gene.

### Item 2

The T cell according to item 1, wherein the recombinant receptor is a chimeric antigen receptor (CAR).

### Item 3

The T cell according to item 1, wherein the recombinant receptor is a chimeric antigen receptor (CAR) that specifically recognizes a tumor antigen.

### Item 4

The T cell according to item 3, wherein the tumor antigen is CD19.

### Item 5

The T cell according to any one of items 1 to 4, wherein the T cell is selected from the group consisting of a CD3+ T cell, a CD8+ T cell, a CD4+ T cell, an immune effector cell, a natural killer (NK) T cell, a γδ T cell, a combination of a CD4+ cell and a CD8T+ cell, a memory cell, a cytokine-induced killer cell, a tumor-infiltrating lymphocyte, and a combination thereof.

### Item 6

The T cell according to item 1, wherein the cell further comprises: an inhibitor for the PRDM1 gene; and at least one of an inhibitor for the RCOR1 gene and an activator for the c-Jun gene.

### Item 7

The T cell according to item 6, wherein
the inhibitor for the PRDM1 gene is the following (1) or (2):
(1) a gene editing system that targets one or more sites inside the PRDM1 gene or a regulatory element of the PRDM1 gene; or
(2) a nucleic acid that encodes one or more components of the gene editing system.

### Item 8

The T cell according to item 6, wherein
the inhibitor for the RCOR1 gene is the following (1) or (2):
(1) a gene editing system that targets one or more sites inside the RCOR1 gene or a regulatory element of the RCOR1 gene; or
(2) a nucleic acid that encodes one or more components of the gene editing system.

### Item 9

The T cell according to item 6, wherein the activator for the c-Jun gene is integration of the c-Jun gene into a cellular DNA via a c-Jun-expressing virus or vector.

### Item 10

A pharmaceutical composition for treating a disease in a subject, the pharmaceutical composition comprising the T cell according to item 1.

### Item 11

The pharmaceutical composition according to item 10, wherein the disease is a tumor or a cancer.

### Item 12

The pharmaceutical composition according to item 10, wherein the T cell maintains an early memory phenotype and performs enhanced or maintained cytokine production, has reduced expression of an exhaustion marker, and/or exhibits suppressed reduction in an effector function as compared to a control T cell that satisfies neither the (I) nor the (II).

### Item 13

A method for enhancing a therapeutic efficacy of a cell that expresses a recombinant receptor that specifically recognizes a tumor antigen, the method comprising:
reducing or eliminating expression and/or function of the PRDM1 gene in the T cell; and
reducing or eliminating expression and/or function of the RCOR1 gene in the T cell, and/or increasing or activating expression of the c-Jun gene in the T cell.

### Item 14

A method for enhancing a therapeutic efficacy of a T cell that expresses a recombinant receptor that specifically recognizes a tumor antigen, the method comprising:
bringing the T cell into contact with an inhibitor for the PRDM1 gene; and
bringing the T cell into contact with an inhibitor for the RCOR1 gene and/or bringing the T cell into contact with an activator for the c-Jun gene.

### Item 15

Use of an inhibitor for the PRDM1 gene and an inhibitor for the RCOR1 gene and/or an activator for the c-Jun gene in manufacturing of a pharmaceutical for a CAR-expressing cell therapy.

### Advantageous Effects of Invention

The present invention provides T cells that maintain high cytotoxicity while having long-term viability. A pharmaceutical composition comprising such T cells and a treatment method of using such T cells are useful for improving T cell-based treatment.

### Brief Description of Drawings

Fig. 1 shows search for an H3K9 methylation-related epigenetic factor influencing differentiation of memory T cells. The data are representative flow cytometry plots. Peripheral blood T cells were stimulated with K562/mOKT3/CD80 (zeroth day) and transduced through a CD19-targeting CAR, and the indicated genes were individually destroyed by using the CRISPR/Cas9 system. The profiles of memory markers were analyzed on the 15^{th} day.
Fig. 2 shows search for an H3K9 methylation-related epigenetic factor influencing differentiation of memory T cells. The profiles of memory markers were analyzed on the 15^{th} day. (A) shows the mean fluorescence intensities of CD62L, (B) shows the mean fluorescence intensities of CCR7, and (C) shows the frequencies (%) of CD62L+CCR7+ cells in a CD8+ CAR-T cell population (in (C), n=3, one-way analysis of variance involving a multiple comparison test through comparison to control CAR-T cells). * P<0.05.

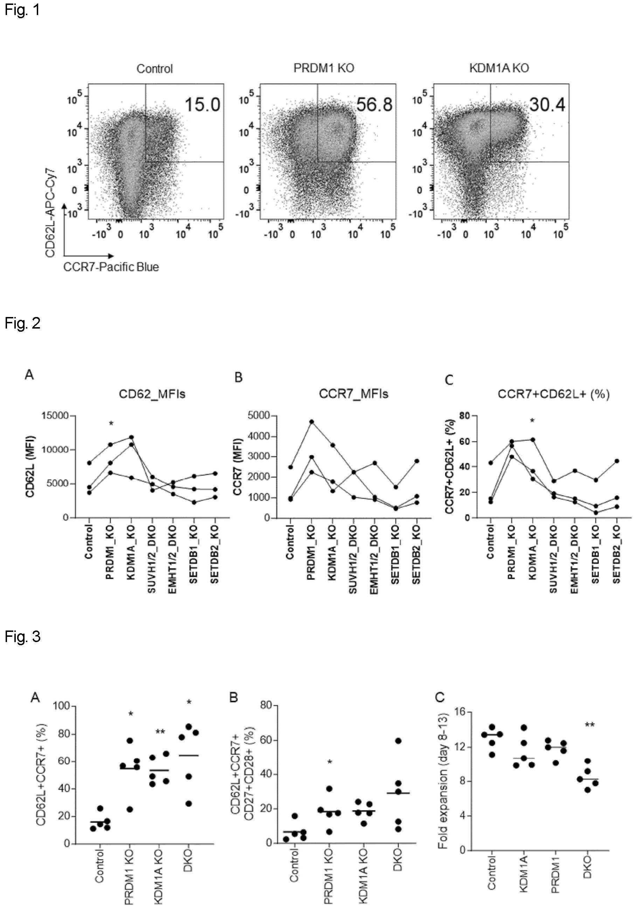

Fig. 3 shows that KDM1A knockout inhibited expansion of T cells. CD19-targeting CAR-T cells were subjected to knockout of PRDM1 and KDM1A individually (PRDM1 KO and KDM1A KO) or simultaneously (DKO). The profiles of memory markers were analyzed from the 11^{th} day to the 13^{th} day. (A) shows the frequencies (%) of CD62L+CCR7+ cells, and (B) shows the frequencies (%) of CD62L+CCR7+CD27+CD28+ cells (in each case, n=5, one-way analysis of variance involving a multiple comparison test through comparison to control T cells). (C) shows fold expansions of CAR-T cells during culturing that took five days (from the eighth day to the 13^{th} day) (n=5, one-way analysis of variance involving a multiple comparison test).
Fig. 4 shows influence of a KDM1A-related factor on differentiation of memory T cells. CD19-targeting CAR-T cells were subjected to knockout of PRDM1 alone or were subjected to knockout of PRDM1 and RCOR1, and respective memory markers were analyzed.
Fig. 5 shows influence of the KDM1A-related factor on differentiation of memory T cells. CD19-targeting CAR-T cells were subjected to knockout of the indicated epigenetic genes. (A) shows the calculated frequencies of CD62L+CCR7+ cells, and (B) shows the calculated frequencies of CD62L+CCR7+CD27+CD28+ cells. In each case, n=6, one-way analysis of variance involving a multiple comparison test. * p<0.05, ** p<0.01.
Fig. 6 shows results of research on influence on expansion of CD19-targeting CAR-T cells, with the CD19-targeting CAR-T cells having been subjected to knockout of the indicated epigenetic genes. Fold expansions during culturing that took five days (from the eighth day to the 13^{th} day) were calculated. n=5, one-way analysis of variance involving a multiple comparison test.
Fig. 7 shows cytotoxicity of CAR-T cells. CD19-targeting CAR-T cells were cocultured overnight with K562 (K562-CD19) for expressing CD19, and surviving K562-CD19 cells were quantified through flow cytometry (n=3, one-way analysis of variance involving a multiple comparison test).

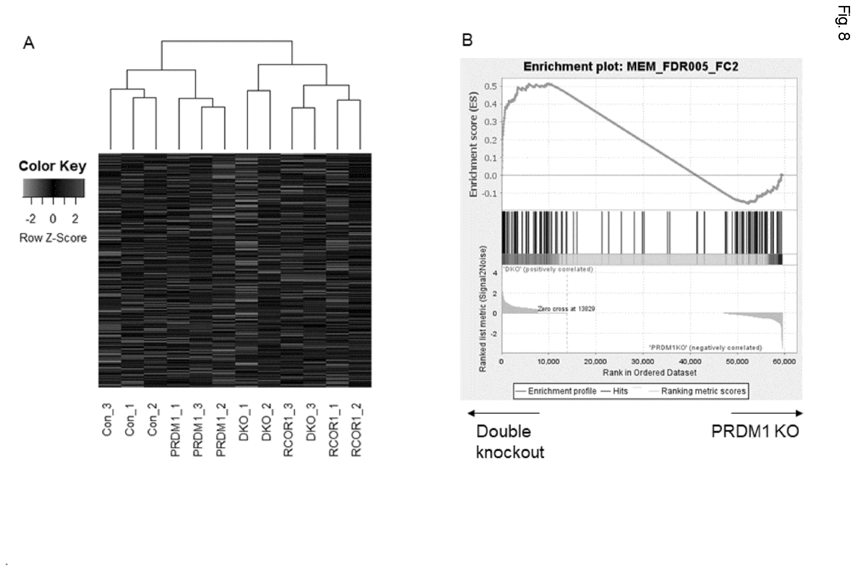

Fig. 8 shows a gene expression profile of knockout CAR-T cells. CD19-targeting CAR-T cells were subjected to gene destruction of PRDM1, RCOR1, or both genes, and the CD19-targeting CAR-T cells were expanded. All RNAs were isolated from a purified CD8⁺ CAR-T cell population (n=three different donors). (A) shows hierarchical clustering of differential expression genes among four groups (FDR<0.05). (B) shows gene set enrichment analysis in which comparison between PRDM1 knockout CAR-T cells and PRDM1 and RCOR knockout CAR-T cells was performed by using a memory T cell-related gene obtained by searching GSE83978.
Fig. 9 shows comparison in terms of the expression levels of effector transcription factors (TBX21 and RUNX3) and the expression levels of exhaustion molecules (TOX and TOX2) among groups which are a control, PRDM1 knockout CAR-T cells, RCOR1 knockout CAR-T cells, and double knockout CAR-T cells. For each group, n=3, paired t-test.
Fig. 10 shows influences inflicted by epigenetic modification on the antitumor effect of CAR-T cells. NALM-6 (NALM6-GL) for expressing EGFP and luciferase was intravenously administered to NSG mice, and each of the NSG mice was treated by using CD19-targeting CAR-T cells in which PRDM1, RCOR1, or both PRDM1 and RCOR1 were knocked out. Representative images showing mice of the respective groups. The mice were continuously monitored in terms of progression of leukemia and the persistence of the injected CAR-T cells.
Fig. 11 shows quantitative total fluxes in the mice at the indicated time points during the treatment in Fig. 10 (for each group, n=8). One-way analysis of variance (ANOVA) involving a multiple comparison test with respect to a logarithmically transformed value.
Fig. 12 shows quantitative total fluxes in the mice at the indicated time points during the treatment in Fig. 10 (for each group, n=8).
Fig. 13 shows analysis of the frequencies of human cells in peripheral bloods in the respective groups in the second, third, fourth, and sixth weeks. One-way analysis of variance involving a multiple comparison test.
Fig. 14 shows overall survival rates of the treated mice (log-rank test).

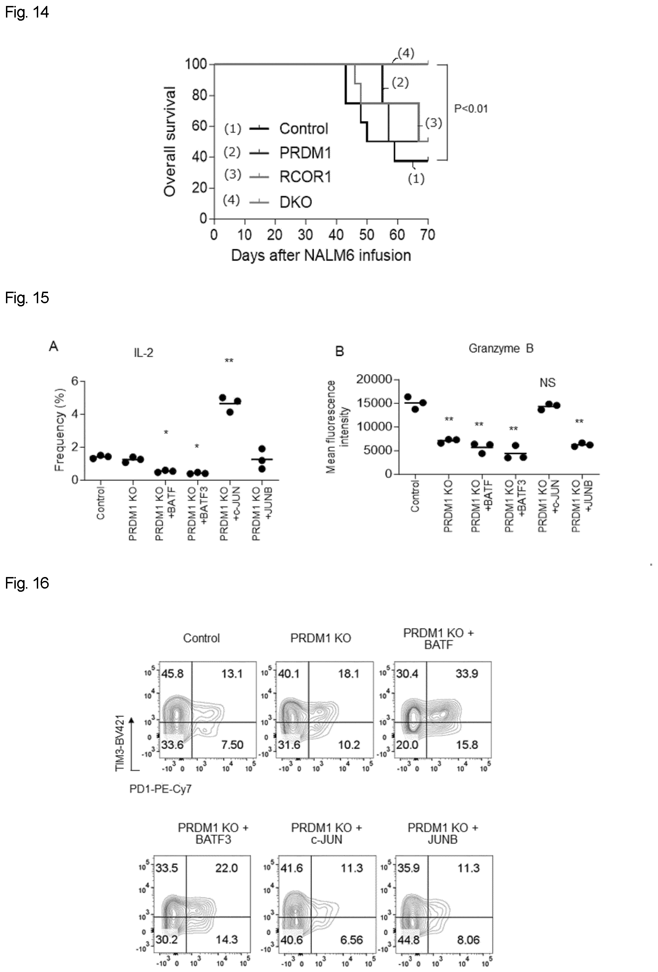

Fig. 15 shows roles of AP1 transcription factors (BATF, BATF3, C-JUN, and JUNB) in PRDM1 knockout CAR-T cells. T cells were individually transduced through a GD2-targeting CAR and the AP1 transcription factors. PRDM1 of the CAR-T cells was deleted, and then the CAR-T cells were re-stimulated with NAL6-GD2 and were expanded for one week. (A) shows analysis of production of IL-2 by the CAR-T cells upon the re-stimulation with the NALM6-GD2 through intracellular flow cytometry (n=3, one-way analysis of variance involving a multiple comparison test through comparison to control CAR-T cells). (B) shows analysis of production of granzyme B by the CAR-T cells upon the re-stimulation with the NALM6-GD2 through intracellular flow cytometry (n=3, one-way analysis of variance involving a multiple comparison test through comparison to the control CAR-T cells).
Fig. 16 shows analysis of exhaustion markers of expanded CAR-T cells. Representative flow cytometry plots.
Fig. 17 shows analysis of the exhaustion markers of the expanded CAR-T cells. Mean fluorescence intensities of PD1, LAG3, and TIM3.
Fig. 18 shows analysis of memory markers of the expanded CAR-T cells. Representative flow cytometry plots.
Fig. 19 shows analysis of the memory markers of the expanded CAR-T cells. The frequency of CD62L+CCR7+ cells (left) or CD62L+CCR7+CD27+CD28+ cells (right) in the CD8⁺ CAR-T cell population (n=3, one-way analysis of variance involving a multiple comparison test through comparison to control CAR-T cells). * P<0.05, ** P<0.01, NS (accidental).
Fig. 20 shows knockout of PRDM1 and overexpression of C-JUN in mesothelin-targeting CAR-T cells. The T cells were transduced through a mesothelin-targeting CAR and C-JUN. PRDM1 of the CAR-T cells was deleted, and then the CAR-T cells were re-stimulated with K562-mesothelin one time or every day, i.e., a total of seven times. Production of granzyme B by the CAR-T cells upon the re-stimulation with the K562-mesothelin was analyzed (n=3, a Student's t-test between the CAR-T cells stimulated one time and the CAR-T cells stimulated seven times).
Fig. 21 shows analysis performed on the CAR-T cells re-stimulated every day in relation to cytokine production through flow cytometry (n=3, one-way analysis of variance involving a multiple comparison test through comparison to control CAR-cells).
Fig. 22 shows analysis of memory markers after re-stimulation performed every day (n=3, one-way analysis of variance involving a multiple comparison test through comparison to control CAR-cells). ** P<0.01; NS stands for "Not Significant".
Fig. 23 shows knockout of PRDM1 and overexpression of C-JUN in GD2-targeting CAR-T cells. The GD2-targeting CAR-T cells were subjected to ectopic expression of C-JUN and were subjected to gene destruction of PRDM1. The CAR-T cells were re-stimulated three times with NALM6-GD2 and were analyzed in relation to production of granzyme B. n=3, one-way analysis of variance involving a multiple comparison test. ** P<0.01; NS stands for "Not Significant".
Fig. 24 shows analysis of exhaustion markers. n=3, one-way analysis of variance involving a multiple comparison test. ** P<0.01; NS stands for "Not Significant".
Fig. 25 shows analysis of cytokine production. n=3, one-way analysis of variance involving a multiple comparison test. ** P<0.01; NS stands for "Not Significant".
Fig. 26 shows analysis of the profiles of memory markers. n=3, one-way analysis of variance involving a multiple comparison test. ** P<0.01; NS stands for "Not Significant".
Fig. 27 shows a gene expression profile of the GD2-targeting CAR-T cells. Hierarchical clustering of genes differing in expression among PRDM1 knockout CAR-T cells regardless of presence or absence of overexpression of C-JUN (log FC>1, FDR<0.01). As a result, significant changes were observed in expression of 959 genes. Importantly, classification of samples in the heat map is not derived from donors and is based on modification of genes. Furthermore, the PRDM1 KO + C-JUN group indicates a clear gene expression profile.
Fig. 28 shows logarithmically transformed counts-per-million reads of the indicated genes (n=3, one-way analysis of variance involving a multiple comparison test). * P<0.05, ** P<0.01.
Fig. 29 shows the frequencies (%) of CD62L+CCR7+ cells in PRDM1/RCOR1 double knockdown CAR-T cells and control knockdown CAR-T cells before antigen stimulation (left) and after antigen stimulation (right).
Fig. 30 shows NOG mice six weeks after non-gene-modified cells (NGMC), the control knockdown CAR-T cells, and the PRDM1/RCOR1 double knockdown CAR-T cells were injected.
Fig. 31 shows quantitative total fluxes in the mice after the control knockdown CAR-T cells (left) and the PRDM1/RCOR1 double knockdown CAR-T cells (right) were injected (respectively n=4 and n=3).
Fig. 32 shows the frequencies (%) of human CD45+ cells in peripheral bloods two weeks after the non-gene-modified cells (NGMC), the control knockdown CAR-T cells, and the PRDM1/RCOR1 double knockdown CAR-T cells were injected (the bar on the left side of each graph) and six weeks after this injection (the bar on the right side of each graph).

### Description of Embodiments

### Definitions

As used herein, singular forms (a, an, and the) each encompass a singular number and a plural number, unless otherwise noted expressly herein or unless an obvious contradiction occurs in terms of context.

As used herein, the verb "comprise" conceptually encompasses "consist essentially of" and "consist of".

The terms "host", "subject", and "patient" are interchangeably used herein to each refer to an individual being treated (through, for example, administration) with the T cells, the pharmaceutical composition, and the method of the present invention. Examples of the subject include, but are not limited to, mammals (e.g., humans, mice, rats, monkeys, horses, cows, pigs, dogs, cats, and the like).

The term "T cell exhaustion" refers to loss of a T cell function, that can occur as a result of a disease. T cell exhaustion is associated with expression of PD-1, TIM-3, and LAG-3, increase in apoptosis, and reduction in secretion of a cytokine. Therefore, the terms "amelioration of T cell exhaustion", "inhibition of T cell exhaustion", "reduction in T cell exhaustion", and the like each refer to a state of a restored function of the T cells characterized by one or more of: reduction in expression and/or level of one or more of PD-1, TIM-3, and LAG-3; increase in formation of memory cells and/or maintenance of a memory marker (e.g., CD62L); prevention of apoptosis; increase in production and/or secretion of an antigen-induced cytokine (e.g., IL-2); enhancement of killing ability; increase in recognition of a tumor target having a low surface antigen; and enhancement of expansion that occurs in response to an antigen.

The terms "cancer" and "tumor" each refer to a tissue or a generated substance containing cells that have lost the ability to regulate growth and expansion. In general, cancer and tumor cells are characterized by loss of contact inhibition, can be invasive, and can exhibit metastasis ability. The present invention is not limited by the type of cancer or the type of treatment, and various cancers including brain cancer or other cancers of the central nervous system, melanoma, lymphoma, bone cancer, epithelial cancer, breast cancer, ovarian cancer, endometrial cancer, colorectal cancer, lung cancer, renal cancer, melanoma, kidney cancer, prostate cancer, sarcoma, carcinoma, and/or a combination thereof can be treated by the T cells, the pharmaceutical composition, and the method according to this description.

The term "effective amount" refers to an amount of the T cells, the pharmaceutical composition, an anticancer drug, or another drug that is effective in terms of a dose and a time period necessary for achieving a desired treatment or preventive effect (e.g., mitigation of some or all of symptoms of a disease being treated).

As used herein, the term "therapeutically effective amount" refers to an amount of a therapeutic agent that is sufficient for causing amelioration of one or more symptoms of a disorder, preventing progression of a disorder, or causing regression of a disorder. For example, one embodiment in the case of treatment of a cancer is as follows. That is, the therapeutically effective amount refers to an amount of a therapeutic agent at which the speed of growth of the tumor is reduced (e.g., tumor burden on the patient is reduced and/or eliminated), the mass of the tumor is reduced, the number of metastases is reduced, progression of the tumor is reduced, or the survival time is increased, by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%.

The term "purification" refers to removal of a contaminant or an undesirable compound from a sample or a composition. As used herein, the term "substantially purified" refers to removal of a contaminant or an undesirable compound from a sample or a composition by about 70 to 90% or by up to 100%.

The terms "administration" and "administering" refer to an act of supplying the T cells or the pharmaceutical composition to a subject. Examples of routes for administration into the body of a human include, but are not limited to, through an eye (ocular), the mouth (oral), the skin (percutaneous), the nose (nasal), a lung (inhalation), the oral mucosa (buccal), an ear, the rectum, by injections (e.g., intravenous, subcutaneous, intraperitoneal, intratumoral, and the like), locally, and the like. In an embodiment, administration of the T cells of the present invention is performed through intravenous injection.

The terms "co-administration" and "co-administering" refer to administration of at least two drugs (e.g., genetically modified immune cells and one or more other drugs exemplified by an anticancer drug) or therapeutic agents into a subject. In several embodiments, co-administration of two or more drugs or therapeutic agents is simultaneous. In another embodiment, a first drug/therapeutic agent is administered before a second drug/therapeutic agent. In several embodiments, the co-administration can be performed through identical or different routes for administration. A person skilled in the art understands that preparations and/or routes for administration of such various drugs or therapeutic agents to be used can differ from each other. A dose appropriate for the co-administration can be easily determined by a person skilled in the art. In several embodiments, in the case of co-administering such drugs or therapeutic agents, each of the drugs or therapeutic agents is administered in a dose lower than a dose appropriate in the case of administering the drug or therapeutic agent alone. Therefore, in an embodiment in which co-administration of drugs or therapeutic agents leads to reduction in necessary doses of (one or more) strongly harmful (e.g., toxic) drugs and/or in a case where co-administration of two or more drugs results in sensitization of the subject to a beneficial effect of one of the drugs owing to co-administration of another one of the drugs, the co-administration is particularly desirable.

As used herein, the term "pharmaceutically acceptable" refers to a composition that substantially does not cause any harmful reaction (e.g., toxic, allergic, or another immunological reaction) when being administered into a subject.

As used herein, the term "pharmaceutically acceptable carrier" refers to any of standard pharmaceutical carriers that include, but are not limited to, phosphate buffered saline solutions, water, various types of humectants (e.g., sodium lauryl sulfate), any and all solvents, dispersion media, coating agents, sodium lauryl sulfate, isotonic and absorption delaying agents, disintegrants (e.g., potato starch and sodium starch glycolate), polyethylene glycol, and the like. The pharmaceutical composition can also contain a stabilizer and a preservative. Examples of carriers, stabilizers, and adjuvants have been described and are publicly known in this technical field (see, for example, Martin, Remington's Pharmaceutical Sciences, 15th Ed., Mack Publ. Co., Easton, Pa. (1975) incorporated herein by reference).

The term "pharmaceutically acceptable salt" refers to any salt (obtained through, for example, a reaction with an acid or a base) of the pharmaceutical composition of the present invention, the salt being physiologically acceptable in a target subject. The "salt" of the pharmaceutical composition of the present invention can be derived from an inorganic or organic acid and base. Examples of the acid include, but are not limited to, hydrochloric acid, hydrobromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, ethanesulfonic acid, formic acid, benzoic acid, malonic acid, sulfonic acid, naphthalene-2-sulfonic acid, benzenesulfonic acid, and the like. Examples of the base include, but are not limited to, alkaline metal (e.g., sodium) hydroxides, alkali earth metal (e.g., magnesium) hydroxides, ammonia, compounds expressed with a formula NW₄+ (in the formula, W represents a C1-4 alkyl), and the like.

Examples of the salt include, but are not limited to, acetate, adipate, alginate, aspartate, benzoate, benzenesulfonate, bisulfate, butyrate, citrate, camphorate, camphorsulfonate, cyclopentanepropionate, digluconate, dodecyl sulfate, ethanesulfonate, fumarate, flucoheptanoate, glycerophosphate, hemisulfate, heptanoate, hexanoate, chloride, bromide, iodide, 2-hydroxyethanesulfonate, lactate, maleate, methanesulfonate, 2-naphthalenesulfonate, nicotinate, oxalate, pamoate, pectate, persulfate, phenylpropionate, picrate, pivalate, propionate, succinate, tartrate, thiocyanate, tosylate, undecanoate, and the like. Other examples of the salt include anions of a compound of the present invention compounded with appropriate cations such as Na+, NH₄+, and NW₄+ (in the formula, W represents a C1-4 alkyl group). For therapeutic use, the salt of the compound of the present invention is meant to be pharmaceutically acceptable. However, salts of acids and bases that are pharmaceutically unacceptable might also be found to be usable for, for example, preparing or purifying a pharmaceutically acceptable compound.

For therapeutic use, the salt contained in the pharmaceutical composition of the present invention is meant to be a pharmaceutically acceptable salt. However, salts of acids and bases that are pharmaceutically unacceptable might also be found to be usable for, for example, preparing or purifying a pharmaceutically acceptable pharmaceutical composition.

As used herein, the term "kit" refers to any delivery system for delivering a material. In the context of an immunotherapeutic agent, examples of the delivery system include a system that enables preservation, transportation, or delivery of an immunogenic factor and/or a support material (e.g., written instructions describing how to use the material, or the like) from a certain position to another position. For example, the kit includes one or more enclosures (e.g., boxes) containing a relevant immunotherapeutic agent (e.g., modified T cells and/or support material). As used herein, the term "fragmented kit" refers to a delivery system including two or more separate containers each containing a constituent sub-portion of the entire kit. The containers may be delivered to an intended recipient together or separately. For example, a first container may contain pharmaceutical compositions including an immunotherapeutic composition for a specific use, and meanwhile, a second container may contain a second drug (e.g., a chemotherapeutic agent). In fact, any delivery system including two or more separate containers each containing a constituent sub-portion of the entire kit is encompassed in the term "fragmented kit". In contrast, a "combined kit" refers to a delivery system containing all of constituents necessary for a specific use inside one container (e.g., one box for accommodating each of such desired constituents). The term "kit" encompasses both the fragmented kit and the combined kit.

The term "immunoglobulin" or "antibody" refers to proteins that bind to one or more epitopes on a specific antigen. Examples of the immunoglobulin include, but are not limited to, polyclonal antibodies, monoclonal antibodies, chimeric antibodies, humanized antibodies, and Fab fragments and F(ab')₂ fragments of the following classes: IgG, IgA, IgM, IgD, IgE, and secretory immunoglobulin (sIg). In general, an immunoglobulin contains two identical heavy chains and two light chains. However, the terms "antibody" and "immunoglobulin" also encompass single-chain antibodies and double-chain antibodies.

A "variable region" or a "variable domain" of the antibody refers to an amino-terminal domain of a heavy chain or a light chain of the antibody. The variable domain of the heavy chain may be referred to as "VH". The variable domain of the light chain may be referred to as "VL". In general, these domains are the most variable portions of the antibody and contain antigen-binding sites.

"Single-chain Fv" or "scFv" antibody fragments include the VH and V domains of the antibodies, and these domains are present in a single polypeptide chain. In general, such a scFv polypeptide further includes, between the VH domain and the VL domain, a polypeptide linker that enables the scFv to form a structure for binding to an antigen.

The term "antigen-binding protein" refers to a protein that binds to a specific antigen. Examples of the "antigen-binding protein" include, but are not limited to: immunoglobulins including polyclonal antibodies, monoclonal antibodies, chimeric antibodies, and humanized antibodies; Fab fragments, F(ab')₂ fragments, and Fab expression libraries; and single-chain antibodies.

As used herein, the term "epitope" refers to a portion of an antigen that makes contact with a specific immunoglobulin.

As used in relation to an interaction between an antibody or a portion thereof (e.g., scFv) and a protein or a peptide, the terms "specific bond" and "specifically binding" mean that the interaction is dependent on presence of a specific sequence or structure (e.g., an antigenic determinant or an epitope) on the protein, i.e., the antibody or the portion thereof (e.g., scFv) recognizes and binds to the specific protein sequence or structure instead of proteins in general. For example, in a case where an antibody is specific to epitope "A", presence of a protein containing epitope A (or free unlabeled A) in a reaction including labeled "A" and the antibody leads to reduction in the amount of the labeled A that binds to the antibody.

As used herein, the term "subject suspected of having a cancer" refers to a subject that exhibits one or more symptoms indicative of a cancer (e.g., a noticeable lump or mass) or that is being screened for a cancer (e.g., during a routine physical examination). The subject suspected of having a cancer may also have one or more risk factors that may develop a cancer. In general, a subject suspected of having a cancer has not been tested for cancer. However, the "subject suspected of having a cancer" encompasses an individual that has received a preliminary diagnosis (e.g., a CT scan showing a mass) but that has not been subjected to a confirmatory test (e.g., a biopsy and/or histology) or for which the type and/or disease stage of the cancer is not known. This term further encompasses a human who has previously had a cancer (e.g., an individual in remission). A "subject suspected of having a cancer" is sometimes diagnosed with the cancer but is sometimes found to have no cancer.

The term "subject diagnosed with a cancer" refers to a subject that has been tested and found to have cancerous cells. The cancer can be diagnosed by employing any appropriate method that includes, but is not limited to, a biopsy, X rays, a blood test, or the like.

The terms "treatment", "therapeutic use", and "medical use" refer to any and all uses of the T cells, the pharmaceutical composition, and the method of the present invention, the uses being for treating a disease or a symptom, or preventing, inhibiting, retarding, or reversing progression of any disease or another undesirable symptom through any method. For example, the terms "treatment of a cancer" and "treatment of a tumor", and grammatical equivalents herein, mean repression, regression, or partial or complete disappearance of a previously developed cancer or tumor. This definition encompasses any reduction in the size, the aggressiveness, or the growth rate of a previously developed cancer or tumor.

The cancer-related terms "improved therapeutic result" and "enhanced therapeutic efficacy" refer to retardation or reduction in growth of cancer cells or a solid tumor, or reduction in the total number of cancer cells or the total tumor burden.

The term "gene delivery system" refers to any means for delivering a pharmaceutical composition including a nucleic acid sequence to a cell or a tissue. Examples of the gene delivery system include, but are not limited to, vectors (e.g., retrovirus, adenovirus, lentivirus, adeno-associated virus, and other nucleic acid-based delivery systems), microinjection of naked nucleic acids, polymer-based delivery systems (e.g., liposome-based and metal particle-based systems), biolistic injection, transduction with use of transposase-based systems for gene integration, Crispr/Cas9-mediated gene integration, non-integrating vectors such as RNA or adeno-associated virus, and the like.

The term "viral gene delivery system" refers to a gene delivery system including viral elements (e.g., intact viruses, modified viruses, and viral components such as nucleic acids or proteins) for facilitating delivery of a sample to a desired cell or tissue. Non-limiting examples of a viral gene delivery system useful for the T cells, the pharmaceutical composition, and the method of the present invention are lentiviral- and retroviral-gene delivery systems.

The term "nucleic acid molecule" refers to, but is not limited to, any nucleic acid-containing molecule including DNA or RNA.

The term "heterologous gene" refers to a gene that is not in the natural environment thereof. Examples of the heterologous gene include genes derived from one species introduced to another species. The heterologous gene also encompasses a gene that is native to an organism modified in a certain manner (e.g., mutated, added into a plurality of copies, linked to a non-native regulatory sequence, or the like). Heterologous genes are distinguished from endogenous genes in that the heterologous gene sequence typically binds to a DNA sequence that is not considered to be naturally associated with a gene sequence in a chromosome or that is associated with a portion of a chromosome not found in nature (e.g., heterologous genes are expressed in gene loci in which the genes are usually not expressed). A cell containing a heterologous gene is described herein as a "modified" or "engineered" cell. For example, a T cell containing a heterologous AP-1 transcription factor gene (e.g., a heterologous AP-1 transcription factor gene expression construct) and/or a heterologous receptor gene (e.g., a heterologous T cell receptor gene expression construct or a heterologous chimeric antigen receptor gene expression construct) is described herein as a modified and/or engineered T cell.

The term "gene expression" refers to a process of converting genetic information encoded in a gene into an RNA (e.g., mRNA, rRNA, tRNA, or snRNA) through "transcription" (i.e., the enzymatic action of an RNA polymerase) of the gene and, for a protein-encoding gene, performing conversion into a protein through "translation" of mRNA. The gene expression can be regulated in many stages during this process. "Up-regulation" or "activation" refers to regulation for increasing production of a gene expression product (i.e., an RNA or a protein), and meanwhile, "down-regulation" or "repression" refers to regulation for reducing the production. Molecules (e.g., transcription factors) involved in the up-regulation and the down-regulation are sometimes referred to as "activator" and "repressor", respectively.

The terms "nucleic acid molecule for encoding", "DNA sequence for encoding", and "DNA for encoding" refer to the order or the sequence of deoxyribonucleotides along the chains of a deoxyribonucleic acid. The order of these deoxyribonucleotides determines the order of amino acids along a polypeptide (protein) chain. Therefore, the DNA sequence encodes the amino acid sequence.

Regarding the term "native protein", the only amino acids contained therein are those observable in the protein when it exists in nature. The native protein can be produced by recombination means or can be isolated from a supply source that exists in nature.

The term "vector" refers to a nucleic acid molecule capable of transporting another nucleic acid to which the vector has bound. One type of vector is "plasmid", which refers to an annular double-stranded DNA into which an additional DNA fragment can be ligated. Another type of vector is phage vector. Another type of vector is viral vector, in which an additional DNA fragment can be ligated into the viral genome. A certain vector is capable of autonomous replication in a host cell into which the vector (e.g., a bacterial vector having a bacterial origin of replication and an episomal mammalian vector) has been introduced. A lentiviral vector or a retroviral vector can be used (in order to, for example, introduce a CAR construct and/or a DNA that encodes one or more AP-1 transcription factors into a cell (e.g., T cell)). Another vector (e.g., a non-episomal mammalian vector) can be integrated into the genome of a host cell upon introduction into the host cell, thereby being replicated together with the host genome. Moreover, a certain vector can induce expression of a gene to which this vector is operatively linked. Such vectors are referred to as "recombinant expression vectors" or simply as "expression vectors" herein. In this description, "plasmid" and "vector" can be interchangeably used since the plasmid is the most commonly used form of vector.

The term "expression vector" refers to a recombinant DNA molecule including an appropriate nucleic acid sequence necessary for expression of a desired coding sequence and an operably linked coding sequence in a specific host organism. Nucleic acid sequences necessary for expression inside prokaryotes usually include promoters, operators (optional), and ribosome binding sites, sometimes in addition to other sequences. Eukaryotic cells are known to utilize promoters, enhancers, and termination and polyadenylation signals.

The term "in vitro" refers to an artificial environment and to a process or a reaction that occurs in the artificial environment. An in vitro environment may be implemented by, but is not limited to, a test tube and a cell culture. The term "in vivo" refers to a natural environment (e.g., an animal or a cell) and to a process or a reaction that occurs in the natural environment.

The term "cell culture" refers to any in vitro culture of cells. This term encompasses continuous cell lines (having, for example, immortal phenotypes), primary cell cultures, transformed cell lines, finite cell lines (e.g., non-transformed cells), and any other cell populations maintained in vitro.

The term "sample" is used in the broadest sense thereof. In one sense, this term is meant to encompass specimens or cultures obtained from any supply sources, and biological and environmental samples. The biological samples can be obtained from animals (including human) and can encompass fluids, solids, tissues, and gases. Examples of the biological samples include blood products such as plasma and serum. However, these examples are not interpreted as limiting the types of samples applicable to the present invention.

The term "knockout (of a gene)" refers to changing a genetic code. Through knockout of a gene, expression and/or function of the gene is completely eliminated.

The term "knockdown (of a gene)" refers to reducing the amount of transcription of the gene. Through knockdown of a gene, expression and/or function of the gene is reduced but is not completely eliminated.

The term "gene editing system" refers to one molecule or two or more molecules that execute modification (e.g., deletion) of one or more nucleic acids at a site of a DNA as a targeted gene or a site near the site. Gene editing systems are publicly known in this technical field.

### Detailed Description of Embodiments of Invention

The present invention is based on a finding that: knockout or knockdown of the PRDM1 gene leads to broad changes in gene expression and the epigenome profile of the T cells and leads to attainment of a profile associated with memory T cells having long-term viability; and knockout or knockdown of the RCOR1 gene or overexpression of the c-Jun gene leads to inhibition of induction of a gene expression profile associated with exhaustion which is a hypofunction state occurring owing to continuous antigen stimulation. Combination of modification of the PRDM1 gene and modification of the RCOR1 gene or the c-JUN gene leads to attainment of a profile in which the features resulting from both modifications are combined.

An aspect of the present invention provides a T cell that expresses a recombinant receptor, the T cell that satisfies the following (I) and (II):
(I) the T cell has reduced or eliminated expression and/or function of the PRDM1 gene; and
(II) the T cell has reduced or eliminated expression and/or function of the RCOR1 gene and/or has increased or activated expression of the c-Jun gene.

In several embodiments, the reduced expression and/or function of the PRDM1 gene in the T cell that expresses a recombinant receptor indicates that knockdown of the PRDM1 gene has led to reduction in expression and/or function of the PRDM1 gene, a transcription product thereof, or a translation product thereof as compared to a control T cell that has not been subjected to knockdown of the PRDM1 gene. The control T cell that has not been subjected to knockdown of the PRDM1 gene may be a T cell that expresses a recombinant receptor and that has yet to be subjected to the knockdown or may be a T cell different from the knocked-down T cell only in that knockdown of the PRDM1 gene is not performed. For example, the reduced expression and/or function of the PRDM1 gene in the T cell that expresses a recombinant receptor refers to being reduced to, for example, 90% or less, 80% or less, 70% or less, 60% or less, 50% or less, 40% or less, 30% or less, 20% or less, or 10% or less of expression and/or function of the PRDM1 gene in the control T cell that has not been subjected to knockdown of the PRDM1 gene, with comparison between these expressions and/or functions being made in terms of the amount of expression of the PRDM1 gene or the activity of the PRDM1 protein.

In several embodiments, the eliminated expression and/or function of the PRDM1 gene in the T cell that expresses a recombinant receptor indicates that knockout of the PRDM1 gene has led to elimination of expression and/or function of the PRDM1 gene, a transcription product thereof, or a translation product thereof.

In several embodiments, the reduced expression and/or function of the RCOR1 gene in the T cell that expresses a recombinant receptor indicates that knockdown of the RCOR1 gene has led to reduction in expression and/or function of the RCOR1 gene, a transcription product thereof, or a translation product thereof as compared to a control T cell that has not been subjected to knockdown of the RCOR1 gene. The control T cell that has not been subjected to knockdown of the RCOR1 gene may be a T cell that expresses a recombinant receptor and that has yet to be subjected to the knockdown or may be a T cell different from the knocked-down T cell only in that knockdown of the RCOR1 gene is not performed. For example, the reduced expression and/or function of the RCOR1 gene in the T cell that expresses a recombinant receptor refers to being reduced to, for example, 90% or less, 80% or less, 70% or less, 60% or less, 50% or less, 40% or less, 30% or less, 20% or less, or 10% or less of expression and/or function of the RCOR1 gene in the control T cell that has not been subjected to knockdown of the RCOR1 gene, with comparison between these expressions and/or functions being made in terms of the amount of expression of the RCOR1 gene or the activity of the RCOR1 protein.

In several embodiments, the eliminated expression and/or function of the RCOR1 gene in the T cell that expresses a recombinant receptor indicates that knockout of the RCOR1 gene has led to elimination of expression and/or function of the RCOR1 gene, a transcription product thereof, or a translation product thereof.

In several embodiments, the increased or activated expression of the c-Jun gene in the T cell that expresses a recombinant receptor indicates that overexpression of the c-Jun gene has led to increase or activation of expression of the c-Jun gene as compared to a control T cell that has not been subjected to overexpression of the c-Jun gene. The control T cell that has not been subjected to overexpression of the c-Jun gene may be a T cell that expresses a recombinant receptor and that has yet to be subjected to overexpression of the c-Jun gene or may be a T cell different from the c-Jun gene-overexpressed T cell only in that overexpression of the c-Jun gene is not performed. For example, the increased or activated expression of the c-Jun gene in the T cell that expresses a recombinant receptor refers to being increased to, for example, 120% or more, 150% or more, or 200% or more of expression of the c-Jun gene in the control T cell that has not been subjected to overexpression of the c-Jun gene, with comparison between these expressions being made in terms of the amount of expression of the c-Jun gene.

"Reduction in the RCOR1 gene" can be used interchangeably with "knockdown of the RCOR1 gene". "Elimination of the RCOR1 gene" can be used interchangeably with "knockout of the RCOR1 gene". Knockdown of the RCOR1 gene leads to reduction in expression and/or function of the RCOR1 gene. Knockout of the RCOR1 gene leads to elimination of expression and/or function of the RCOR1 gene.

The T cell may be a native T cell or may be an engineered T cell (e.g., tumor-infiltrating lymphocyte (TIL)). The T cell that expresses a recombinant receptor may be a T cell that expresses a chimeric antigen receptor (CAR) or a T cell receptor (TCR). The type of the T cell is not limited.

In several embodiments, the T cell is a CD3+ T cell (e.g., a combination of CD4+ and CD8+ T cells). In an embodiment, the T cell is a CD8+ T cell. In an embodiment, the T cell is a CD4+ T cell. In several embodiments, the T cell is a natural killer (NK) T cell. In several embodiments, the T cell is a γδ T cell. In several embodiments, the T cell is a combination of a CD4+ and a CD8T+ cell (e.g., CD3+). In an embodiment, the T cell is a memory T cell. In an embodiment, the T cell is a combination of a CD8+ T cell, a CD4+ T cell, a NK T cell, a memory T cell, and/or a γδ T cell. In several embodiments, the T cell is an immune effector cell. In several embodiments, the T cell is a cytokine-induced killer cell.

In several embodiments, the T cells are selected from the group consisting of a CD3+ T cell, a CD8+ T cell, a CD4+ T cell, an immune effector cell, a natural killer (NK) T cell, a γδ T cell, a combination of a CD4+ cell and a CD8T+ cell, a memory cell, a cytokine-induced killer cell, a tumor-infiltrating lymphocyte, and a combination thereof

The means for genetically expressing a CAR or a TCR in the T cell is not limited, and any means known in this technical field and/or described herein can be employed. Non-limiting examples of the means for genetically engineering the T cells include, but are not limited to: retrovirus- or lentivirus-mediated transduction; transduction with use of transposase-based systems for gene integration; Crispr/Cas9-mediated gene integration; non-integrating vectors such as RNA or adeno-associated virus; and other means described herein.

The T cell of the present invention may have a recombinant receptor including an antigen-binding domain. In several embodiments, the T cell of the present invention may have a chimeric antigen receptor (CAR) including an antigen-binding domain. Selection of the antigen-binding domain is dependent on the type and the number of ligands that define the surface of a target cell. For example, the antigen-binding domain of the CAR can be selected to recognize a ligand that acts as a cell surface marker on a target cell associated with a specific disease. Examples of the cell surface marker that can act as the ligand for the antigen moiety domain in the CAR include cell surface markers associated with viral, bacterial, and parasitic infections, autoimmune diseases, and cancer cells. The CAR can target an intended tumor antigen by, for example, having a desired antigen-binding domain to specifically bind to the antigen on a tumor cell.

In several embodiments, the T cell is a T cell that expresses a recombinant receptor that specifically recognizes a tumor antigen. In another embodiment, the T cell is, for example, a T cell that expresses a chimeric antigen receptor that specifically recognizes a tumor antigen. In another embodiment, the T cell is a peripheral blood-derived T cell genetically modified to express a chimeric antigen receptor that specifically recognizes a tumor antigen.

The tumor antigen is a protein that is produced by a tumor cell and that exhibits immune response (in particular, T cell-mediated immune response). Tumor antigens are known in this technical field. The tumor antigen may include one or more antigenic cancer antigens/epitopes associated with malignant tumors.

The tumor antigen can be a tumor-specific antigen (TSA) or a tumor-associated antigen (TAA). The TSA is unique to tumor cells and is not generated in other cells inside the body. The TAA is not unique to tumor cells, and instead, is expressed also on several normal cells under a condition that an immunological tolerant condition is not induced to the antigen. Expression of an antigen on a tumor can occur under a condition that enables the immune system to respond to the antigen.

Examples of the TSA or the TAA include, but are not limited to: differentiation antigens such as MART-1/Melan-A (MART-1), gp100 (Pmel 17), tyrosinase, TRP-1, and TRP-2; tumor-specific multi-lineage antigens such as MAGE-1, MAGE-3, BAGE, GAGE-1, GAGE-2, and p15; overexpressed embryonic antigens such as CEA; overexpressed oncogenes and mutated tumor-suppressor genes such as p53, Ras, and HER-2/neu; unique tumor antigens resulting from chromosomal translocation such as BCR-ABL, E2A-PRL, H4-RET, IGH-IGK, and MYL-RAR; and viral antigens such as Epstein-Barr virus antigen EBVA and the human papillomavirus (HPV) antigens E6 and E7. Examples of other large protein-based antigens include TSP-180, MAGE-4, MAGE-5, MAGE-6, RAGE, NY-ESO-1, p185erbB-2, p180erbB-3, c-met, nm-23H1, PSA, TAG-72, CA19-9, CA72-4, CAM17.1, NuMa, K-ras, β-catenin, CDK4, Mum-1, p15, p16, 43-9F, 5T4, 791Tgp72, α-fetoprotein, β-HCG, BCA225, BTAA, CA125, CA15-3/CA27.291/BCAA, CA195, CA242, CA-50, CAM43, CD68/P1, CO-029, FGF-5, G250, Ga733/EpCAM, HTgp-175, M344, MA-50, MG7-Ag, MOV18, NB/70K, NY-CO-1, RCAS1, SDCCAG16, PSMA, TA-90/Mac-2 binding protein/cyclophilin C-related protein, TAAL6, TAG72, TLP, and TPS.

In several embodiments, the tumor antigen is selected from the group consisting of CD5, CD19, CD20, CD22, CD33, CD38, CD44v6, CD47, CD70, CD116, CD123, CD138, CD269, ROR1, GD2, EBV proteins or antigens, folate receptors, mesothelin, GM2, GPC3, human carcinoembryonic antigens, CD33/IL3Ra, c-Met, PSMA, glycolipid F77, EGFRvIII, NY-E SO-1, MAGE-A3, MART-1, gp100, Her2, p53, α-fetoprotein (AFP), and combinations thereof.

In several embodiments, the chimeric antigen receptor (CAR) expressed in the T cell includes, as an antigen-binding domain, a fusion protein (e.g., a single-chain variable fragment (scFv)) of variable regions of a heavy chain (VH) and a light chain (VL) in an immunoglobulin to specifically bind to a tumor antigen (e.g., GD2).

Any antibody to specifically bind to a tumor antigen (e.g., GD2) or an antibody fragment as a portion of the antibody can be used for constructing an antigen-binding site or an antigen-binding domain of the CAR for expression in immune cells to be used for the treatment method of the present invention. Examples of such an antibody/immunoglobulin against GD2 include, but are not limited to, 14G2a, ch14.18, hu14.18K322A, m3F8, hu3F8-IgG1, hu3F8-IgG4, HM3F8, UNITUXIN, DMAb-20, and any other antibodies to specifically bind to GD2.

The T cell of the present invention may have a recombinant receptor including a transmembrane domain. In several embodiments, the T cell of the present invention may have a chimeric antigen receptor (CAR) including a transmembrane domain. In several embodiments, the tumor antigen CAR may incorporate variable lengths of hinge regions and/or transmembrane domains between antigen-binding domains and signaling domains. The transmembrane domains may encompass, but are not limited to, any transmembrane domains including all or some of TCRζ chain (CD3ζ), CD28, OX40/CD134, 4-1BB/CD137/TNFRSF9, FcERIγ, ICOS/CD278, ILRB/CD122, IL-2RG/CD132, and CD40 transmembrane domains.

The T cell of the present invention may have a recombinant receptor including an intracellular signaling domain for transmitting an event of ligand binding to an intracellular signal that activates the T cell. In several embodiments, the T cell of the present invention may have a chimeric antigen receptor (CAR) including the intracellular signaling domain. The intracellular signaling domain may be, for example, CD3ζ of a native T cell receptor complex and/or another signaling domain (e.g., a MyD88 signaling domain).

In a case where there is no co-stimulatory signal, receptor-ligand binding might be insufficient for satisfactory activation and expansion of the T cell. In view of this, the recombinant receptor (e.g., CAR) expressed in the T cell may include one or more co-stimulatory domains (e.g., for providing an additional signal in order to stimulate the T cell for satisfactory activation). In one embodiment, a co-stimulatory domain that increases CAR immune T cell cytokine production is used. In another embodiment, a co-stimulatory domain that facilitates replication of the T cell is used. In still another embodiment, a co-stimulatory domain that prevents exhaustion of the CAR-T cell is used. In another embodiment, a co-stimulatory domain that increases antitumor activity of the T cell is used. In still another embodiment, a co-stimulatory domain that enhances survival of the CAR-T cell (e.g., after injection into a patient) is used. Examples of a protein that may be used for providing a co-stimulatory signal or a domain or portion of the protein include, but are not limited to: B7-1/CD80; CD28; B7-2/CD86; CTLA-4; B7-H1/PD-L1; ICOS/CD278; ILRB/CD122; IL-2RG/CD132; B7-H2; PD-1; B7-H3; PD-L2; B7-H4; PDCD6; BTLA; 4-1BB/TNFRSF9/CD137; FcERIγ; CD40 ligand/TNFSF5; 4-1BB ligand/TNFSF9; GITR/TNFRSF18; BAFF/BLyS/TNFSF13B; GITR ligand/TNFSF18; BAFFR/TNFRSF13C; HVEM/TNFRSF14; CD27/TNFRSF7; LIGHT/TNFSF14; CD27 ligand/TNFSF7; OX40/TNFRSF4; CD30/TNFRSF8; OX40 ligand/TNFSF4; CD30 ligand/TNFSF8; TACl/TNFRSF13B; CD40/TNFRSF5; 2B4/CD244/SLAMF4; CD84/SLAMF5; BLAME/SLAMF8; CD229/SLAMF3; CD2CRACC/SLAMF7; CD2F-10/SLAMF9; NTB-A/SLAMF6; CD48/SLAMF2; SLAM/CD150; CD58/LFA-3; CD2; Ikaros; CD53; integrin α4/CD49d; CD82/Kai-1; integrin α4β1; CD90/Thy1; integrin α4β7/LPAM-1; CD96; LAG-3; CD160; LMIR1/CD300A; CRTAM; TCL1A; DAP12; TIM-1/KIM-1/HAVCR; dectin-1/CLEC7A; TIM-4; DPPIV/CD26; TSLP; EphB6; TSLP R; and HLA-DR.

In several embodiments, the CAR receptor specific to a tumor antigen includes an antigen-binding domain to specifically bind to the tumor antigen, a transmembrane domain, an intracellular signaling domain, and at least one optionally selected co-stimulatory domain.

The T cell of the present invention may further include: an inhibitor for the PRDM1 gene; and at least one of an inhibitor for the RCOR1 gene and an activator for the c-Jun gene.

In several embodiments, the inhibitor for the PRDM1 gene is any of the following (i) to (v):
(i) (1) a gene editing system that targets one or more sites inside the PRDM1 gene or a regulatory element of the PRDM1 gene, (2) a nucleic acid that encodes one or more components of the gene editing system, or (3) a combination of (1) and (2);
(ii) an siRNA or an shRNA specific to the PRDM1, or a nucleic acid that encodes the siRNA or the shRNA;
(iii) a protein or small molecule;
(iv) a dominant negative binding partner of a protein encoded by the PRDM1 gene, or a nucleic acid that encodes the dominant negative binding partner; or
(v) a dominant negative mutant of the protein encoded by the PRDM1 gene, or a nucleic acid that encodes the dominant negative mutant.

Inclusion of such an inhibitor for the PRDM1 gene enables the T cell to have reduced or eliminated expression and/or function of the PRDM1 gene.

In several embodiments, the gene editing system is selected from the group consisting of CRISPR/Cas9 systems, zinc-finger nuclease systems, TALEN systems, and meganuclease systems. The gene editing system can bind to any target sequence of the PRDM1 gene, e.g., an exon, an intron, 5'UTR, or 3'UTR. In several embodiments, the gene editing system is a CRISPR/Cas system containing a gRNA molecule including a targeting sequence that hybridizes to the target sequence of the PRDM1 gene.

In several embodiments, the inhibitor for the RCOR1 gene is any of the following (i) to (v):
(i) (1) a gene editing system that targets one or more sites inside the RCOR1 gene or a regulatory element of the RCOR1 gene, (2) a nucleic acid that encodes one or more components of the gene editing system, or (3) a combination of (1) and (2);
(ii) an siRNA or an shRNA specific to the RCOR1, or a nucleic acid that encodes the siRNA or the shRNA;
(iii) a protein or small molecule;
(iv) a dominant negative binding partner of a protein encoded by the RCOR1 gene, or a nucleic acid that encodes the dominant negative binding partner; or
(v) a dominant negative mutant of the protein encoded by the RCOR1 gene, or a nucleic acid that encodes the dominant negative mutant.

Inclusion of such an inhibitor for the RCOR1 gene enables the T cell to have reduced or eliminated expression and/or function of the RCOR1 gene.

In several embodiments, the gene editing system is selected from the group consisting of CRISPR/Cas9 systems, zinc-finger nuclease systems, TALEN systems, and meganuclease systems. In several embodiments, the gene editing system can bind to any target sequence of the RCOR1 gene, e.g., an exon, an intron, 5'UTR, or 3'UTR. In several embodiments, the gene editing system is a CRISPR/Cas system containing a gRNA molecule including a targeting sequence that hybridizes to the target sequence of the RCOR1 gene.

In several embodiments, the activator for the c-Jun gene is any of the following (i) to (vi):
(i) integration of the c-Jun gene into a cellular DNA via a c-Jun-expressing virus or vector;
(ii) a small molecule or a protein that regulates expression of the c-Jun;
(iii) integration of the c-Jun gene into a cellular DNA via a CRISPR/Cas9-based system;
(iv) expression of the c-Jun gene via an RNA or an oncolytic virus, or a transient expression system;
(v) ex vivo delivery of the c-Jun into the T cell for adoptive transfer; and
(vi) delivery of the c-Jun into the T cell through in vivo gene delivery.

Inclusion of such an activator for the c-Jun gene enables the T cell to have increased or activated expression of the c-Jun gene.

The c-Jun is one of AP-1 transcription factors induced after activation of the T cell and is associated with production and secretion of a cytokine (e.g., interleukin-2) by the T cell. The T cell that expresses a CAR experiences tonic antigen-independent signaling by receptor clustering and indicates basic biology about T cell exhaustion, such as one indicated by a high level of PD-1, TIM-3, and LAG-3 expression, reduced production of an antigen-induced cytokine, and excessive programmed cell death. The c-Jun is identified as a substance to be reduced in exhausted T cells. Meanwhile, overexpression of the c-Jun gene inhibits T cell exhaustion to prevent or suppress a T cell dysfunction associated with T cell exhaustion.

In several embodiments, the activator for the c-Jun gene is (i) integration of the c-Jun gene into a cellular DNA via a c-Jun-expressing virus or vector, and the c-Jun can be expressed from an expression construct such as the virus or the vector through a method that is known in genetic engineering. In several embodiments, the chimeric antigen receptor and the c-Jun are expressed from different expression constructs, and at least one of these expression constructs is a viral vector. In several embodiments, the chimeric antigen receptor and the c-Jun are co-expressed from a single expression construct, and this expression construct is a viral vector.

In several embodiments, the c-Jun gene may be a mutated c-Jun gene or a cleaved c-Jun gene (a portion of the c-Jun gene). The c-Jun can be mutated and/or cleaved without the ability of the mutated/cleaved c-Jun being influenced by mediation of rescue of a dysfunctional T cell. The T cell can be modified to have increase or activated expression of such a mutated c-Jun gene or such a cleaved c-Jun gene. For example, a c-Jun polypeptide having N-terminal deletion and mutation maintains the ability thereof to rescue functions of HA-28z exhausted CAR-T cells and keep equivalent increase in cytokine production as compared to the native c-Jun. In several embodiments, the c-Jun polypeptide includes mutation or deletion causing transactivation and/or inactivation or disappearance of a δ domain. In several embodiments, the mutated/cleaved c-Jun polypeptide has a sequence identity of 70% or more (e.g., 70%, 75%, 80%, 85%, 90%, 95%, 99%, 100%, or a value in any of the ranges therebetween) of C-terminal amino acid residues (e.g., 50 residues, 75 residues, 100 residues, 150 residues, 200 residues, 250 residues, or a value in any of the ranges therebetween), a C-terminal portion (e.g., one fourth, one third, or one half of the entire length of an amino acid), or a C-terminal domain (e.g., ε, bZIP and the amino acid C-terminal thereof) of wild-type c-Jun. In several embodiments, N-terminal amino acid residues (e.g., 50 residues, 75 residues, 100 residues, 150 residues, or a value in any of the ranges therebetween), an N-terminal portion (e.g., one fourth, one third, one half), or an N-terminal domain (e.g., δ, a transactivation domain, and the amino acid N-terminal thereof) of the wild-type c-Jun is deleted, mutated, or inactivated.

The T cell of the present invention may be further modified to reduce and/or eliminate expression and/or activity of one or more AP-1 inhibitory complex members. Examples of the AP-1 inhibitory complex members include Jun B, BATF family members (e.g., BATF3), IRF4, and ATF family members. As described above in relation to the inhibitor for the PRDM1 gene and the inhibitor for the RCOR1 gene, such modification can be performed by utilizing technologies known in this technical field, such as the CRISPR/Cas9 system, zinc-finger nuclease targeting, or expression of TALEN, siRNA, shRNA, a protein inhibitor (e.g., a chemical inhibitor, a small molecule inhibitor, an antibody, or the like), or a dominant negative form.

The T cell of the present invention may be further modified to inhibit activity of a drug (PD1, PD-L1, CTLA4, TIM3, CEACAM, LAG3, VISTA, BTLA, TIGIT, LAIR1, CD160, 2B4, TGFβ, or the like) that inhibits activity of the T cell. For example, the T cell may be modified to have an intracellular domain of this drug.

In several embodiments, the T cell according to any of the above embodiments of the present invention maintains an early memory phenotype and performs enhanced or maintained cytokine production, has reduced expression of an exhaustion marker, and/or exhibits suppressed reduction in an effector function as compared to a control T cell that satisfies neither the following (I) nor (II).

(I) the T cell has reduced or eliminated expression and/or function of the PRDM1 gene; and
(II) the T cell has reduced or eliminated expression and/or function of the RCOR1 gene and/or has increased or activated expression of the c-Jun gene.

In several embodiments, the T cell according to any of the above embodiments of the present invention maintains the early memory phenotype and exhibits suppressed reduction in the effector function as compared to a PRDM1 single knockout cell.

In several embodiments, the maintenance of the above early memory phenotype is evaluated based on the frequency of early memory T cells. In several embodiments, the maintenance of the above early memory phenotype is evaluated based on expression of a gene (e.g., IL7R, TCF7, CCR7, or MYB) characteristic of memory T cells. For example, when the proportion of the early memory T cells among T cells after expansion of the T cell (in particular, CAR-T cell) that expresses a recombinant receptor is not less than 2 times the proportion of the early memory T cells before the expansion, the early memory phenotype is considered to be maintained.

In several embodiments, the cytokine production is production of IL-2, IFN-γ, and/or TNF-α.

In several embodiments, the exhaustion marker comprises PD1 and/or TIM3. In several embodiments, the exhaustion marker comprises TOX and/or TOX2.

In several embodiments, the suppression of the reduction in the effector function is evaluated based on expression of an effector transcription factor (e.g., TBX21 or RUNX3). In several embodiments, the suppression of the reduction in the effector function is evaluated based on expression of a gene (PDCD1 or granzyme B) characteristic of an inexhaustible effector T cell.

Another aspect of the present invention provides a pharmaceutical composition for treating a disease in a subject, the pharmaceutical composition comprising the T cell according to any of the above embodiments of the present invention.

In several embodiments, the disease is a tumor or a cancer.

The pharmaceutical composition may comprise a pharmaceutically acceptable carrier. The pharmaceutical composition may further comprise one or more other drugs (e.g., chemotherapeutic drugs). The pharmaceutical composition may optionally comprise one or more additional drugs such as another drug for treating T cell exhaustion (e.g., an anti-PD-1 checkpoint inhibitor such as nivolumab) or another drug to be used for treating a subject for a disease associated with T cell exhaustion (e.g., an antiviral agent, an antibiotic, an antibacterial agent, or an anticancer drug).

Unlike monoclonal antibodies which do not efficiently pass through the blood-brain barrier, activated CAR-T cells efficiently enter the central nervous system after being adoptively transferred.

Another aspect of the present invention provides a disease treatment method comprising administering an effective amount of any of the above T cells of the present invention to a subject having a disease. The present invention is not limited by the type of the disease being treated. Any disease capable of being treated by administration of the T cells can be treated in a more effective manner through the method of using any of the above T cells of the present invention and/or the pharmaceutical composition comprising any of the above T cells of the present invention. In one embodiment, the disease is a cancer or a tumor. The present invention is not limited by the type of the cancer or the tumor. In one embodiment, administration of the effective amount of any of the above T cells of the present invention to a subject having a disease enables long-term viability of the T cells and inhibits T cell exhaustion (as compared to, for example, the case of administering the same amount of unmodified T cells to the subject) in this subject. For example, in an embodiment, this method comprises a method for treating a subject having a disease responsive to treatment utilizing an adoptive cell therapy. In an embodiment, the method for treating a subject having a disease responsive to treatment utilizing an adoptive cell therapy comprises administering the effective amount of the pharmaceutical composition comprising any of the above T cells of the present invention, and this method enables long-term viability of the T cells and inhibits T cell exhaustion (as compared to, for example, the case of administering the same amount of unmodified T cells to the subject) in this subject.

An embodiment provides a method in which a cancer and/or a tumor is treated (e.g., inhibited from growing and/or killed) by using any of the above T cells of the present invention, the T cells (e.g., CD3+ T cells) being engineered to transmit a signal for activating the T cells so as to: express a receptor that recognizes a tumor surface antigen (e.g., CD19, CD20, CD22, ROR1, GD2, or EBV protein or antigen, folate receptor, mesothelin, human carcinoembryonic antigen, CD33/IL3Ra, c-Met, PSMA, glycolipid F77, EGFRvIII, NY-ESO-1, MAGE-A3, MART-1, gp100, and/or p53); and induce expansion of the T cells and/or tumor killing. Non-limiting examples of the receptor include a chimeric antigen receptor (CAR) into which an antibody binding domain (e.g., scFv derived from a monoclonal antibody) that recognizes a tumor surface antigen (e.g., GD2), a transmembrane domain (e.g., a transmembrane domain of CD8), and one or more intracellular signaling domains are integrated.

An embodiment of the present invention provides a method for treating a cancer or retarding progression of the cancer in a subject, the method comprising administering the effective amount of any of the above T cells of the present invention to the subject. In several embodiments, this treatment generates continuous response in the individual after stoppage of the treatment. Another embodiment of the present invention provides a method for enhancing an immune function in a subject having a cancer, the method comprising administering the effective amount of any of the above T cells of the present invention to the subject.

In several embodiments, the subject has a cancer resistant to one or more other types of anticancer therapies (e.g., chemotherapy, immunotherapy, and the like). In several embodiments, the resistance involves recurrence or intractability of the cancer. The recurrence can refer to post-therapy reemergence of the cancer at the site of origin or a new site. In several embodiments, the resistance involves progression of the cancer during treatment through chemotherapy. In several embodiments, the resistance involves unresponsiveness of the cancer to conventional therapies in which chemotherapeutic agents are used. The cancer can be resistant at the time of start of treatment or can become resistant during the treatment. In several embodiments, the cancer is in an early stage or a late stage.

In an embodiment, an immunotherapeutic composition comprising the T cell of the present invention genetically modified to express a tumor-specific CAR is used for treating, ameliorating, or preventing a cancer (e.g., cancer cells having chemoresistance, radiation resistance, hormone resistance, or the like) characterized by being resistant to one or more conventional cancer therapies.

In an embodiment, any of the above T cells of the present invention, an immunotherapeutic composition comprising the T cells, and a method of using the T cells can be employed for inducing cytotoxicity to tumor cells and/or promoting survival and function of immune cells. For example, any of the above T cells of the present invention, the immunotherapeutic composition comprising the T cells, and the method of using the T cells can be employed for: inducing interleukin-2 (IL-2) for promoting T cell survival; inducing Fas ligand and/or tumor necrosis factor-related apoptosis-inducing ligand (TRAIL); and/or inducing interferon γ. In several embodiments, any of the above T cells of the present invention, the immunotherapeutic composition comprising the T cells, and the method of using the T cells are employed for inducing cell cycle arrest and/or apoptosis and/or intensifying induction of cell cycle arrest and/or apoptosis, either alone or in response to an additional apoptosis-inducing signal. In several embodiments, any of the above T cells of the present invention, the immunotherapeutic composition comprising the T cells, and the method of using the T cells sensitize cancer cells to induction of cell cycle arrest and/or apoptosis, the cancer cells including cells usually resistant to such an inductive stimulus.

In several embodiments, any of the above T cells of the present invention, a pharmaceutical composition comprising the T cells, and the method of using the T cells are employed for treating cells, a tissue, or an organ inflicted with a disease, a pathological condition of the disease, and/or the disease in an animal (examples thereof include, but are not limited to, mammal patients including humans and companion animals). In several embodiments, the cancer cells being treated are metastatic. In another aspect, the cancer cells being treated are resistant to anticancer drugs.

Any of the above T cells of the present invention or the pharmaceutical composition comprising the T cells may be used in combination with at least one additional therapeutic agent (examples thereof include, but are not limited to, anticancer drugs, apoptosis regulators, antibacterial agents, antiviral agents, antifungal agents, and anti-inflammatory agents) in a method for treating a disease in a subject.

Any of the above T cells of the present invention or the pharmaceutical composition comprising the T cells may be used in combination with a radiation therapy in a method for treating a disease in a subject.

The T cells according to any of the embodiments of the present invention are contained in an amount effective in achieving the intended purpose thereof. Although individual needs differ, determination of an optimum range for the effective amount of each constituent is within the technical scope in this technical field. In one non-limiting example, the T cells according to any of the embodiments of the present invention may be administered into a mammal (e.g., human) in order to provide 1000 to 10¹⁰ T cells per day to the human (e.g., in order to treat a cancer). In another embodiment, 1000 to 10¹⁰ modified T cells are administered for treating, ameliorating, or preventing a cancer (e.g., preventing metastasis, recurrence, and/or progression of the cancer). A unit dose of the T cells may be administered one or more times per day in one or more administrations (e.g., for one, two, three, four, five, or six days or weeks or longer).

The T cells may be administered as a portion of a pharmaceutical formulation comprising an appropriate pharmaceutically acceptable carrier containing an excipient and an aid that facilitate processing and/or administration of the modified cells into the pharmaceutically usable formulation. The T cells and/or the pharmaceutical formulation comprising these T cells may be administered in an intravenous, intramuscular, subcutaneous, intratumoral, intraperitoneal, intrathecal, or intracardiac ventricular manner. The T cells and/or the pharmaceutical formulation comprising these T cells may be administered in an effective amount in order to prevent or treat a disease. An appropriate dose may be determined based on the type of the disease being treated, the type of the modified T cells, the severity and course of the disease, a clinical condition of the individual, a clinical history and response to treatment of the individual, and determination by a primary care physician.

The efficacy of any of the methods described herein (e.g., treatment in which the T cells according to any of the embodiments of the present invention is used alone or used in combination with one or more of the chemotherapeutic agents described herein) can be tested by various means known in this technical field such as a clinical or preclinical model. An appropriate preclinical model will be presented herein as an example. For any exemplary model, tumors are developed, and then the mice are randomly sorted into treatment groups for receiving treatment or control treatment. Tumor sizes (e.g., tumor volumes) are measured during the course of treatment, and the overall survival rate is also monitored.

Another aspect of the present invention provides a method for enhancing a therapeutic efficacy of a T cell having a chimeric antigen receptor (CAR) or a T cell receptor (TCR), the method comprising:
reducing or eliminating expression and/or function of the PRDM1 gene in the T cell; and
reducing or eliminating expression and/or function of the RCOR1 gene in the T cell, and/or increasing or activating expression of the c-Jun gene in the T cell.

In several embodiments, the step of reducing or eliminating expression and/or function of the PRDM1 gene in the T cell includes administering the above inhibitor for the PRDM1 gene to the T cell to reduce or eliminate expression and/or function of the PRDM1 gene.

In several embodiments, the step of reducing or eliminating expression and/or function of the RCOR1 gene in the T cell, and/or increasing or activating expression of the c-Jun gene in the T cell includes administering the above inhibitor for the RCOR1 gene to the T cell to reduce or eliminate expression and/or function of the RCOR1 gene.

In several embodiments, the step of reducing or eliminating expression and/or function of the RCOR1 gene in the T cell, and/or increasing or activating expression of the c-Jun gene in the T cell includes administering the above activator for the c-Jun gene to the T cell to increase or activate expression of the c-Jun gene.

In several embodiments, the step of reducing or eliminating expression and/or function of the RCOR1 gene in the T cell, and/or increasing or activating expression of the c-Jun gene in the T cell includes: administering the above inhibitor for the RCOR1 gene to the T cell to reduce or eliminate expression and/or function of the RCOR1 gene; and administering the above activator for the c-Jun gene to the T cell to increase or activate expression of the c-Jun gene.

Another aspect of the present invention provides a method for enhancing a therapeutic efficacy of a T cell having a chimeric antigen receptor (CAR) or a T cell receptor (TCR), the method comprising:
bringing the T cell into contact with an inhibitor for the PRDM1 gene; and
bringing the T cell into contact with an inhibitor for the RCOR1 gene and/or bringing the T cell into contact with an activator for the c-Jun gene.

Another aspect of the present invention provides use of an inhibitor for the PRDM1 gene and an inhibitor for the COR1 gene and/or an activator for the c-Jun gene in manufacturing of a pharmaceutical for a CAR-expressing cell therapy.

Therefore, the T cells according to the above embodiments of the present invention, the pharmaceutical composition comprising the T cells, and the method of using the T cells can be useful for treating any disease using the T cells since the T cells have undergone knockout or knockdown of the PRDM1 gene to have long-term viability and have undergone knockout or knockdown of the RCOR1 gene or overexpression of the c-Jun gene to exhibit inhibited induction to exhaustion, which is a hypofunction state occurring owing to continuous antigen stimulation, and to have improved cytotoxicity. In particular, the present technologies are generally applicable to adoptive immunotherapies against cancers. In a genetically modified T cell therapy with use of CAR-T cells, TCR-T cells, or the like or a TIL therapy with use of tumor-infiltrating T cells, genetic modification is performed during in vitro culturing or expansion, whereby antitumor T cells capable of post-infusion induction of a persistent antitumor effect can be produced. These technologies are applicable irrespective of the types of target antigens and applicable cancers.

The disclosures of all patent applications and literatures cited herein are construed as being entirely incorporated herein by reference.

Examples described below are intended to be taken merely as examples and are not intended to limit the technical scope of the present invention in any way. Reagents were commercially available or were obtained or prepared according to methods commonly employed in this technical field or procedures in publicly-known literatures, unless otherwise specified.

### Examples

### Materials and Methods

### Cell Line

Erythroleukemia cell line K562 was obtained from the cell bank (Osaka, Japan) of the Japanese Collection of Research Bioresources (JCRB). CD19⁺B cell leukemia cell line NALM6 was obtained from the Cell Resource Center for Biomedical Research of Tohoku University (Sendai, Japan). K562-OKT3/CD80 was produced through retroviral transduction of CD80 and scFV derived from an anti-CD3 monoclonal antibody (Clone OKT3) to which a transmembrane domain and a cytoplasmic domain of CD8α were linked (Yoshikawa, T. et al. Blood 139, 2156-2172 (2022)). NALM6-GL was prepared through retroviral transduction of the NALM6 by EGFP linked to codon optimized luciferase (Luc2) via a 2A peptide sequence derived from porcine teschovirus-1 (P2A). GD2 expressed NALM6-GL was produced by introducing a GM2/GD2 synthase and a GD3 synthase into the NALM6-GL. The K562, the NALM6, and the derivatives thereof were cultured in 10% FBS-added RPMI-1640 (nacalai tesque, Inc., Kyoto, Japan). PG13 cells were cultured in 10% FBS-added DMEM (manufactured by nacalai tesque, Inc.).

### Human T Cell Culture

Peripheral blood mononuclear cells (PBMC) (Cellular Technology Limited, Cleveland, Ohio) derived from healthy donors were stimulated according to the above-described report (Yoshikawa, T. et al. Blood 139, 2156-2172 (2022)) at an effector-to-target ratio of 7:1 by using the K562-OKT3/CD80 having been subjected to mitomycin C treatment. The stimulated T cells were cultured in an RPMI-1640 culture medium containing 10% fetal bovine serum, 1% penicillin/streptomycin, and recombinant IL-2 (100 IU/mL, NIPRO CORPORATION, Osaka, Japan).

In order to produce CAR-T cells, two days after the T cells were stimulated by using RetroNectin (Takara Bio Inc., Kusatsu, Japan), a CD19-targeting CAR gene, a mesothelin-targeting CAR gene, or a GD2-targeting CAR gene was subjected to retroviral transduction into the T cells. PG13 packaging cells were used for retrovirus production. CAR constructs were each obtained by linking a single-chain variable fragment (scFv) derived from clone FMC63 (targeting CD19) (Nicholson, I. C. et al. Immunol 34, 1157-1165 (1997)), ss1 (targeting mesothelin) (Li, Q., Verschraegen et al., Anticancer Res 24, 1327-1335 (2004)), or 14g2a (targeting GD2) (Richman, S. A. et al., Cancer Immunol Res 6, 36-46 (2018)) having an E101K high-affinity variant to CD28 and CD3z signaling domains. K562-CD19 or NALM-6, K562-mesothelin, and NALM6-GD2 cells were used for re-stimulating the CD19-targeting CAR-T cells, the mesothelin-targeting CAR-T cells, and the GD2-targeting CAR-T cells, respectively.

### Gene Knockout via CRISPR/Cas9

Knockout via CRISPR/Cas9 was performed by using the Alt-R CRISPR/Cas9 system available from Integrated DNA Technologies, Inc. (IDT, Coralville, IA) (Yoshikawa, T. et al. Blood 139, 2156-2172 (2022)). Electroporation of a ribonucleoprotein (RNP) complex was performed by using NEPA 21 Electroporator (NEPA GENE CO., LTD., Ichikawa, Japan) after the T cells were stimulated. Chemically synthesized crRNA and tracrRNA (IDT) were mixed, and the resultant mixture was annealed at 95°C for five minutes and then gradually cooled to room temperature. Next, Alt-R Cas9 Nuclease V3 (25 µg, IDT) was incubated at 37°C for 10 to 15 minutes together with the annealed guide RNA (300 pmol) to generate an RNP complex. Before electroporation, Alt-R Cas9 Electroporation Enhancer was added at a final concentration of 2 µM. The following parameters were used in the electroporation. That is, regarding a poring pulse, the voltage was 275 V, the pulse length was 1 msec, the pulse interval was 50 msec, the number of pulses was 2, and the attenuation rate was 10%, with a polarity of +. Regarding a transfer pulse, the voltage was 20 V, the pulse length was 50 msec, the pulse interval was 50 msec, the number of pulses was ±5, and the attenuation rate was 40%, with polarities of ±.

A genome DNA was extracted after 48 to 72 hours by using the NucleoSpin DNA Rapid Lyse kit (MACHEREY-NAGEL GmbH & Co. KG, Dueren, Germany). A genomic region including the target site was amplified by PCR, and the amplicon was used for determining a Sanger sequence. The gene-editing efficiency of each gRNA was calculated through Inference of CRISPR Edits (ICE) analysis (Conant, D. et al. CRISPR J 5, 123-130 (2022)).

### Flow Cytometry Analysis

Flow cytometry analysis was performed by using LSRFortessa (Becton, Dickinson and Company, Franklin Lakes, NJ) or CytoFLEX-S (Beckman Coulter, Inc., Brea, CA). Antibodies used were: PerCP/Cyanine5.5 anti-human CD271 (ME20.4, BioLegend, Inc.); APC-anti CD8 (RPA-T8, BioLegend, Inc.); FITC-anti CD8 (RPA-T8, BioLegend, Inc.); FITC-anti CD45RA (Clone HI100, Becton, Dickinson and Company); Pacific blue-anti CCR7 (G043H7, BioLegend, Inc.); APC-Cy7-anti CD62L (DREG-56, BioLegend, Inc.); PE-Cy7-anti CD27 (M-T271, BioLegend, Inc.); PE-anti CD28 (CD28.2, BioLegend, Inc.); FITC-anti IL2 (Clone MQ1-17H12 manufactured by BioLegend, Inc.); PE-Cy7-anti IFNγ (Clone 4S.B3, BioLegend, Inc.); Brilliant Violet 421-anti TNFα (Clone/monoclonal antibody 11, BioLegend, Inc.); Alexa Fluor 647 anti-CD45 (Clone HI30, BioLegend, Inc.); PE-Cy7-anti CD279 (PD1) (Clone EH12.1, Becton, Dickinson and Company); BV421-anti TIM3 (Clone 7D3, Becton, Dickinson and Company); APC-R700-anti LAG-3 (Clone T47-530, Becton, Dickinson and Company); and PE-anti GD2 (Clone 14g2a, BioLegend, Inc., #357304). Data were analyzed by using the software FlowJo v10 (Becton, Dickinson and Company).

The CAR-T cells were cocultured with the indicated target cells and were incubated for six hours in order to analyze cytokine production by the T cells. Two hours after the stimulation, Brefeldin A (BioLegend, Inc.) was added. Thereafter, the cells were fixed and permeabilized by using Cyto-Fas Fix/Perm kit (BioLegend, Inc.) according to the protocol of the manufacturer.

### In Vitro Cytotoxicity Test

1x10⁵ CAR-T cells were cocultured with EGFP⁺ target cells at an indicated ratio in order to evaluate the cytotoxicity of the CAR-T cells. The absolute number of surviving target tumor cells was measured through flow cytometry. The frequency of the surviving tumor cells was calculated as a ratio to the number of cells cultured without the CAR-T cells. Dead cells were distinguished by staining for which LIVE/DEAD Fixable Near-IR Dead Cell Stain kit (Thermo Fisher Scientific Inc.) was used.

### RNA Sequencing Analysis

Paired-end RNA sequencing analysis was performed by using NovaSeq 6000 (Illumina) available from RIKEN GENESIS CO., LTD. (Tokyo, Japan) or Rhelixa, Inc. (Tokyo, Japan). An FASTQ file was subjected to quality trimming and was mapped onto a GRCh38 human reference genome by using Hisat2 (version 2.1.0). The difference in gene expression among groups was identified by using edgeR. Unsupervised hierarchical clustering of the difference in gene expression among the groups was performed by using the heatmap.2 function of the gplots package. Logarithmically transformed counts-per-million reads were used as gene expression levels. GSEA was performed with GSEA v2 (Broad Institute) by using a memory T cell-related gene set extracted from GSE83978 (FDR<0.05, log fold-change>2). All of analyses of count data having been read were performed in the R environment.

### Statistical Analysis

Significance of the difference between two groups was evaluated through an unpaired two-tailed t-test or a paired two-tailed t-test. The differences between three or more groups were compared by utilizing one-way analysis of variance (one-way ANOVA). In the indicated cases, data were compared through non-parametric tests among which: a Mann-Whitney test was performed for two groups; and a Kruskal-Wallis test was performed for three or more groups. Survival time analysis in animal experiments was performed through the Kaplan-Meier method, and, for the difference between two groups, a log-rank test was performed. Regarding significant difference, in a case where the p-value was less than 0.05, the difference was regarded as being statistically significant. A p-value less than 0.05 was defined as corresponding to a significant correlation. Software GraphPad Prism 8 was used for statistical analysis and graphic design.

### Results

The present inventors previously proved that gene destruction of PRDM1 promoted maintenance of early memory phenotypes, and as a result, led to improvement of the persistence of the CAR-T cells so that the antitumor effect persisted (Yoshikawa, T. et al. Blood 139, 2156-2172 (2022)). Blimp-1 encoded by PRDM1 forms a transcriptional repressor complex together with a plurality of epigenetic factors and partially regulates chromatin accessibility via histone H3K9 trimethylation modification (Shin, H. M. et al. Immunity 39, 661-675 (2013)). The present inventors examined whether or not other epigenetic factors associated with H3K9 methylation would also influence the differentiation states of memory T cells in human CAR-T cells. As a result of subjecting the CD19-targeting CAR-T cells to knockout of H3K9 methylation-related genes individually, the present inventors found that knockout of KDM1A led to significant increase in the expression levels of early memory markers such as CCR7 and CD62L (Figs. 1 and 2(A) to (C)).

Next, the present inventors thought that simultaneous knockout of PRDM1 and KDM1A might lead to further enhancement of maintenance of early memory phenotypes. However, double knockout of PRDM1 and KDM1A led to prominent inhibition of expansion of T cells without additively increasing the frequency of early memory T cells (Figs. 3(A) to (C)).

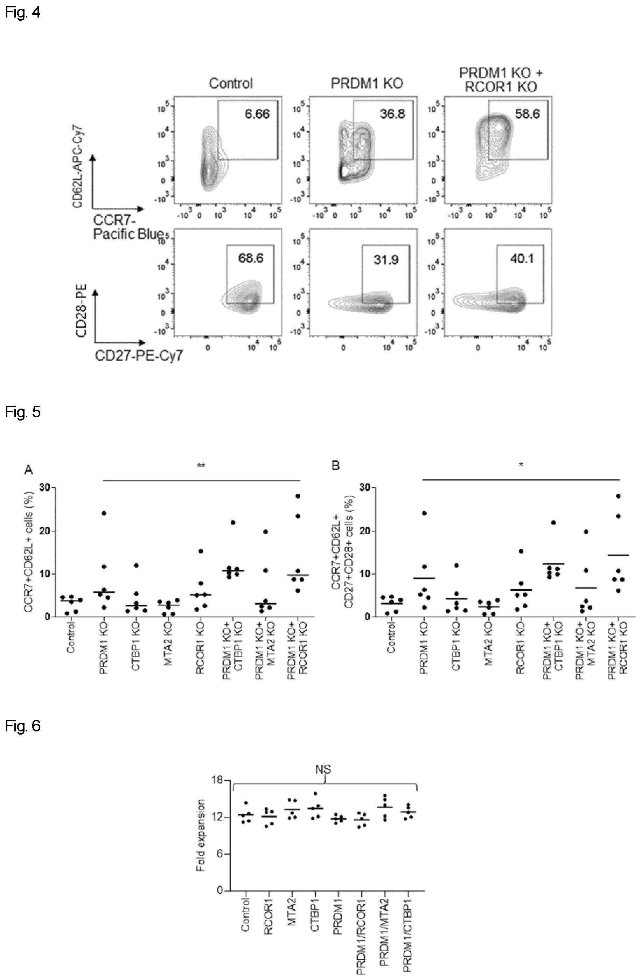

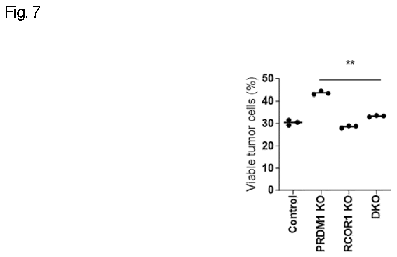

KDM1A is also associated with formation of various transcription complexes mainly including CoREST, NuRD, and CtBP (Lynch, J. T., Expert Opin Ther Targets 16, 1239-1249 (2012)). Considering this, the present inventors examined whether or not destruction of these complexes instead of KDM1A would lead to promotion of formation of memory T cells without inducing toxicity. As shown in Figs. 4, 5(A), and (B), double knockout of PRDM1 and RCOR1 led to significant increase in the frequency of CD62L+CCR7+ cells or CD62L+CCR7+CD27+CD28+ CAR-T cells as compared to knockout of PRDM1 alone. Importantly, knockout of RCOR1 did not lead to repression of expansion of the CAR-T cells (Fig. 6). Unexpectedly, PRDM1/RCOR1 double knockout CAR-T cells also exhibited improvement of cytotoxicity with respect to CD19+ target cells as compared to PRDM1 knockout CAR-T cells (Fig. 7). These results indicate that gene destruction of PRDM1 and RCOR1 could lead to enhancement of the persistent effector function of the CAR-T cells.

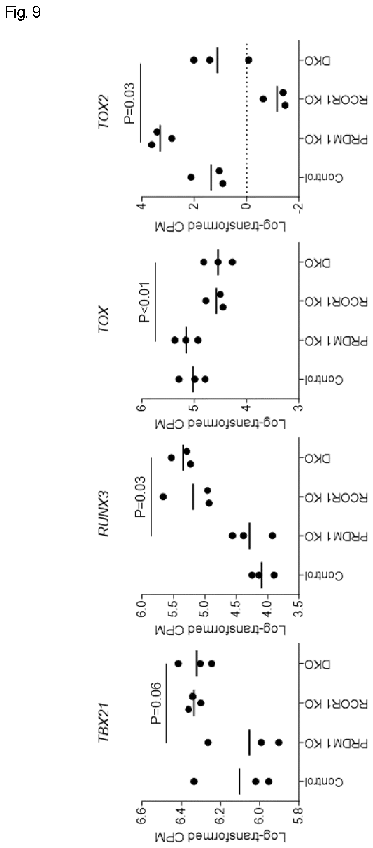

The present inventors compared the gene expression profiles of the PRDM knockout CAR-T cells, RCOR1 knockout CAR-T cells, and double knockout CAR-T cells through RNA sequencing analysis. A control, the PRDM1 knockout CAR-T cells, and the RCOR1 knockout CAR-T cells were separated into different clusters according to hierarchical clustering (FDR<0.05) of differentially expressed genes among four groups, and this separation indicates that PRDM1 and RCOR1 regulate expression of different gene groups (Fig. 8(A)). Through gene set enrichment analysis, it was indicated that, in the double knockout CAR-T cells, genes upregulated by memory T cells were significantly enriched as compared to exhausted T cells obtained by searching GSE83978 (FDE<0.05, log-fold change>2) (Fig. 8(B)). Correspondingly to the improvement of the cytotoxicity, the double knockout CAR-T cells exhibited increase in the expression levels of effector transcription factors such as TBX21 and RUNX3 and exhibited reduction in the expression levels of exhaustion molecules such as TOX and TOX2 as compared to the PRDM1 knockout CAR-T cells (Fig. 9).

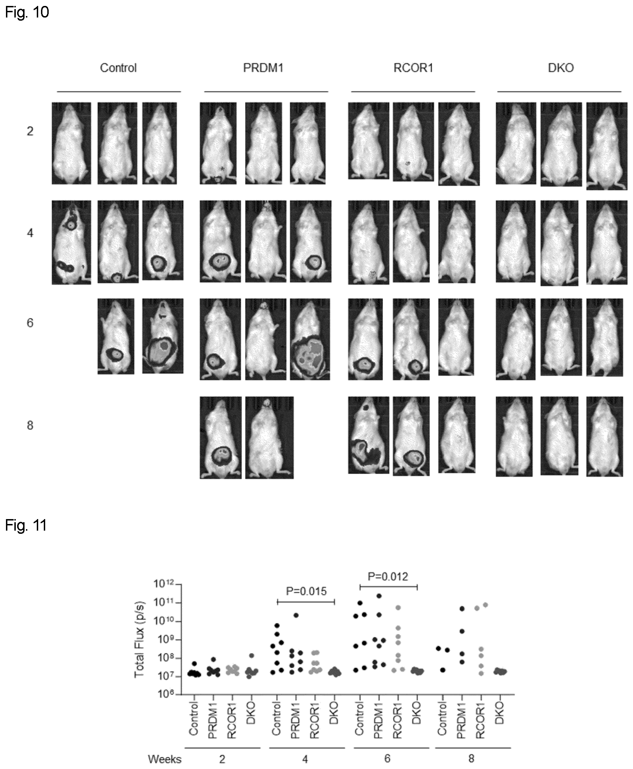

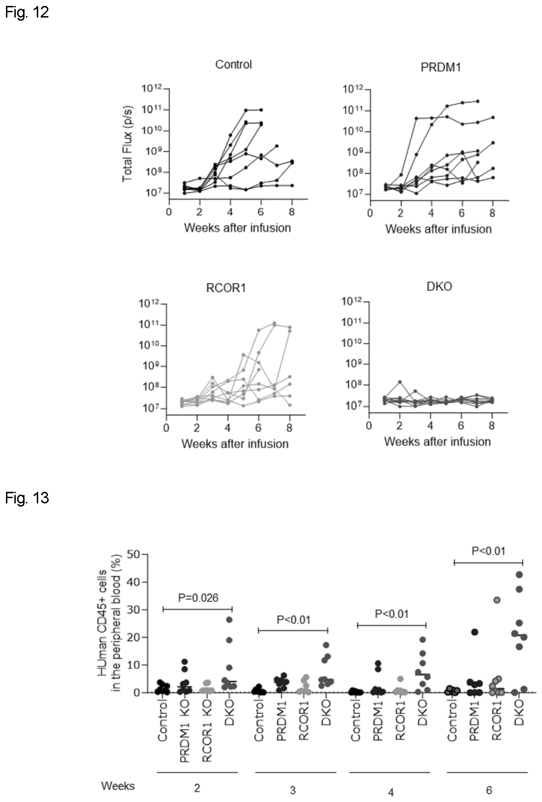

Next, the present inventors examined an in vivo therapeutic efficacy of such PRDM1/RCOR1 double knockout CAR-T cells. CD19-targeting CAR-T cells subjected to or not subjected to knockout of PRDM1 or RCOR1 were injected to each of NSG mice having NALM6. Surprisingly, the double knockout CAR-T cells subjected to knockout of both PRDM1 and RCOR1 exhibited excellent therapeutic efficacy as compared to the control or as compared to the single knockout CAR-T cells each subjected to knockout of one of PRDM1 and RCOR1 (Figs. 10 to 12). Importantly, the double knockout CAR-T cells exhibited the most prominent persistence in peripheral blood (Fig. 13), and as a result, had higher overall survival rates than the other groups (Fig. 14).

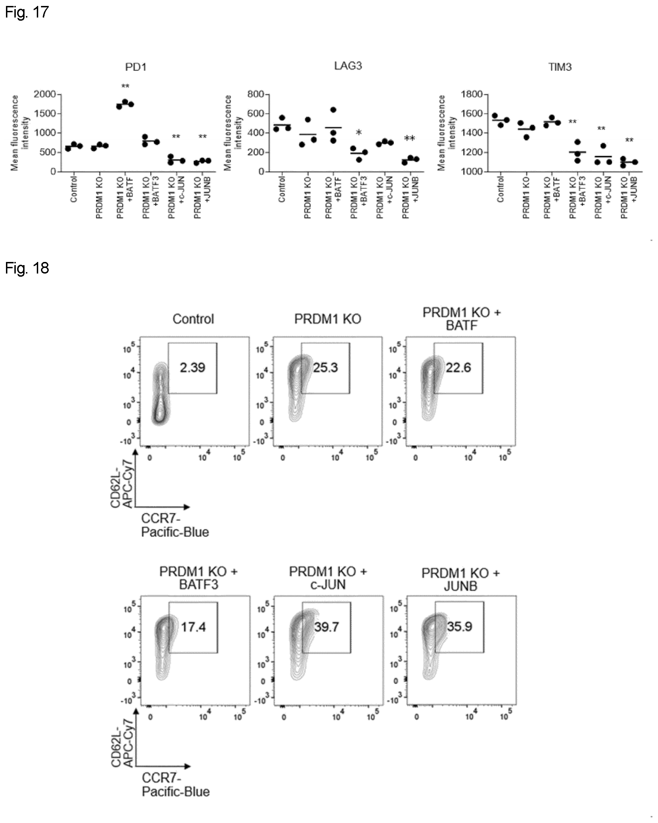

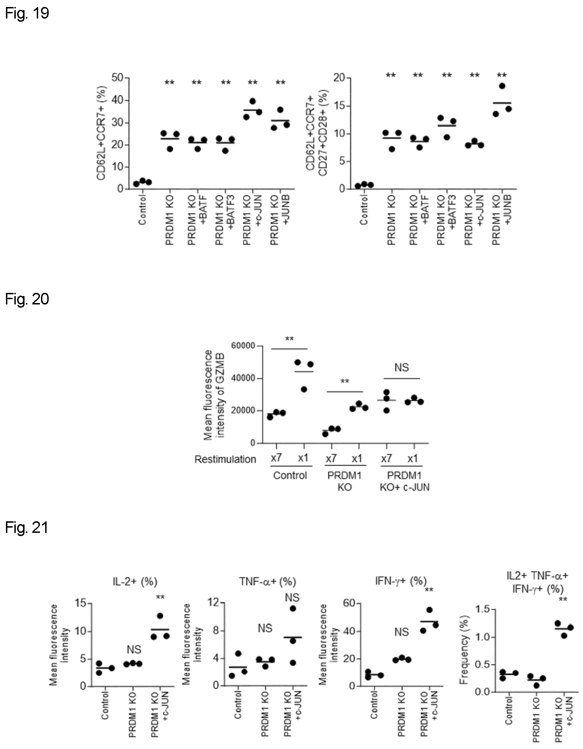

The present inventors further searched for another factor for enhancing the persistence of the effector function of the PRDM1-destroyed CAR-T cells. AP-1 transcription factors (AP1 TFs) play an important role in the function of effector T cells, but the role of the AP-1 transcription factors in induction of T cell exhaustion is controversial. There are studies indicating that AP1 TFs promote T cell exhaustion (Lynch, J. T. et al. Expert Opin Ther Targets 16, 1239-1249 (2012); Man, K. et al. Immunity 47, 1129-1141. e1125 (2017)), and meanwhile, there are also studies indicating that AP1 TFs counteract T cell exhaustion and maintain strong effector functions (Chen, J. et al., Nature 567, 530-534 (2019); Chen, Y. et al., Nat Immunol 22, 996-1007 (2021)). In order to clarify what kind of influence would be inflicted by individual AP1 TFs on functional characteristics of the PRDM1 knockout CAR-T cells, the present inventors used a retroviral vector to ectopically express BATF, BATF3, c-JUN, and JUNB in PRDM1-destroyed anti-GD2-28z CAR-T cells (Long, A. H. et al., Nat Med 21, 581-590 (2015)) known to sustain T cell exhaustion owing to tonic signaling. As shown in Fig. 15(A), overexpression of c-JUN led to significant improvement of production of IL-2 by CD8⁺ CAR-T cells. This improvement is one of characteristics of multifunctional T cells not having been exhausted. Knockout of PRDM1 leads to improvement of characteristics of memory T cells, but meanwhile, leads to impairment of effector functions. Importantly, expression of c-JUN in the PRDM1 knockout CAR-T cells led to restoration of production of granzyme B to the same level as the control CAR-T cells (Fig. 15(B)). In addition, CAR-T cells in which c-JUN was overexpressed exhibited reduction in the level of immune inhibitory molecules such as PD1 and TIM3 (Figs. 16 and 17). None of the AP1 TFs impaired memory phenotypes increased through the knockout of PRDM1 (Figs. 18 and 19).

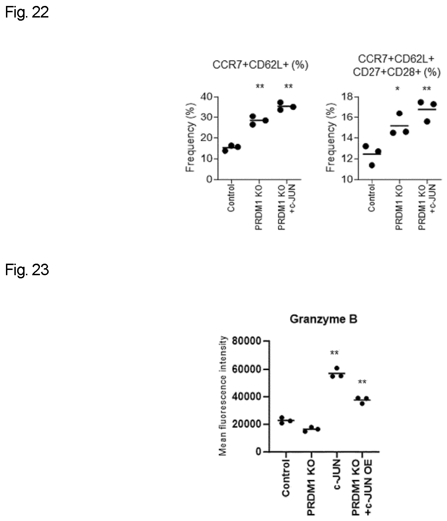

The present inventors also tested strategies of knockout of PRDM1 and overexpression of c-JUN by using different types of CAR-T cells. The mesothelin-targeting CAR-T cells were stimulated with the K562-mesothelin every day, and effector functions of the mesothelin-targeting CAR-T cells were analyzed. It was confirmed that production of granzyme B by control CAR-T cells was significantly attenuated through the repetitive stimulation of the CAR-T cells, and this experimental protocol reproduced induction of T cell exhaustion (Fig. 20). In contrast, ectopic expression of c-JUN in CAR-T cells in which PRDM1 was deleted canceled out reduction in effector function caused by the repetitive stimulation. In addition, the CAR-T cells in which PRDM1 was deleted and c-JUN was overexpressed exhibited enhancement of cytokine production (Fig. 21), and furthermore, retained early memory phenotypes as compared to the control CAR-T cells (Fig. 22).

Furthermore, continuous antigen stimulation by NALM6-GD2 was performed also on the GD2-targeting CAR-T cells, and then effector functions of the GD2-targeting CAR-T cells were analyzed. In contrast to the negative effect on secretion of granzyme B due to knockout of PRDM1, expression of c-JUN led to significant enhancement of production of granzyme B irrespective of the knockout of PRDM1 (Fig. 23).

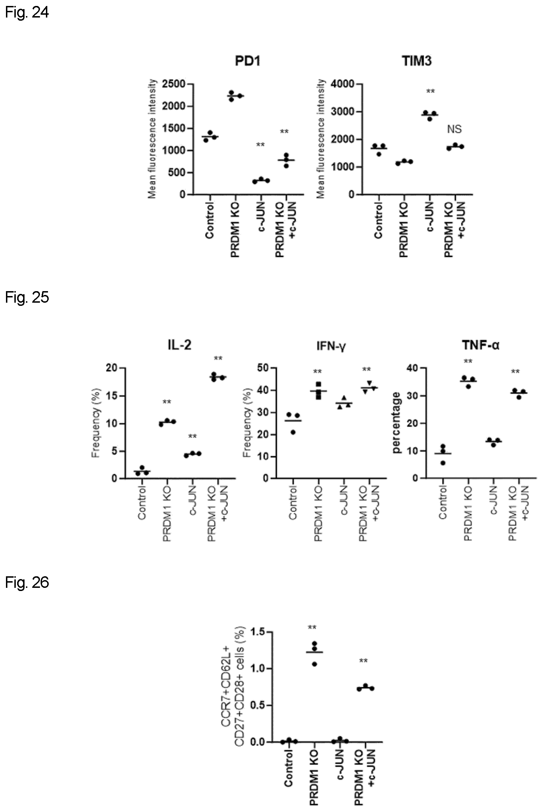

PRDM1/c-JUN double modification CAR-T cells had reduced expression of exhaustion markers (PD1 and TIM3, Fig. 24), exhibited increased cytokine production (Fig. 25), and favorably maintained early memory phenotypes (Fig. 26) as compared to control CAR-T cells. In particular, the amount of IL-2 having been produced exceeded a sum obtained in the case of performing knockout of PRDM1 and overexpression of c-JUN singly.

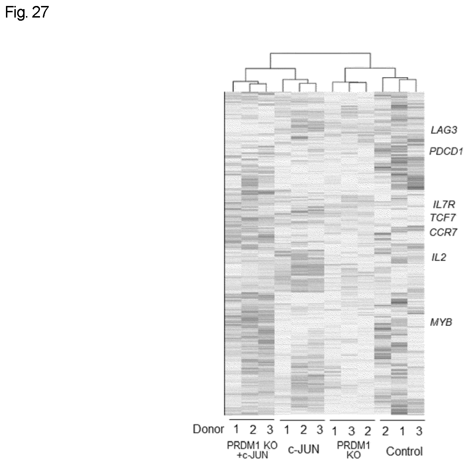

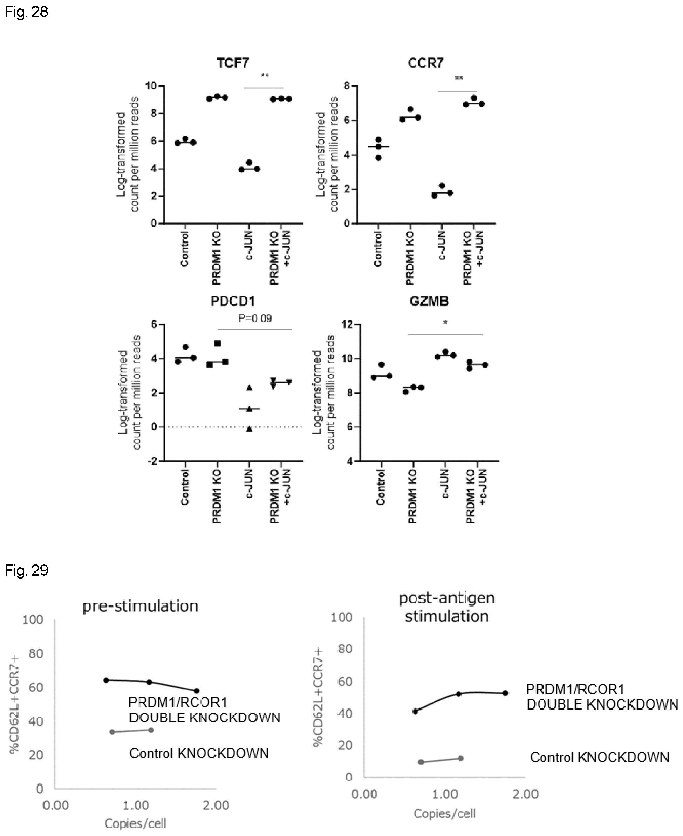

These results indicate that the knockout of PRDM1 and the overexpression of c-JUN achieved both longevity characteristics and strong effector functions. In order to confirm these observations, the gene expression profile of the PRDM1/c-JUN modified anti-GD2 CAR-T cells was analyzed. Hierarchical clustering of expressed genes different from one another provided an indication that the four groups formed different clusters regardless of differences in the donor samples (Fig. 27). The CAR-T cells in which PRDM1 was deleted and c-JUN was expressed attained a gene expression profile which was characteristic of both memory T cells (e.g., increase in the levels of IL7R, TCF7, CCR7, and MYB) and inexhaustible effector T cells (e.g., reduction in the level of PDCD1 and increase in the level of GZMB) (Fig. 28). These results indicate that the CAR-T cells in which PRDM1 was knocked out and c-JUN was expressed attained a long-lived and inexhaustible phenotype necessary for a persistent antitumor effect.

Next, the present inventors examined an in vivo therapeutic efficacy of PRDM1/RCOR1 double knockout siRNA co-expressed mesothelin-targeting CAR-T cells. The siRNA co-expressed mesothelin-targeting CAR-T cells were produced by using a lentiviral vector for human peripheral blood mononuclear cells (PBMC) (manufactured by HemaCare Corporation) derived from healthy donors. A PRDM1/RCOR1 double knockdown vector was provided with two types of siRNA sequences targeting PRDM1 and two types of siRNA sequences targeting RCOR1, and a control knockdown vector was provided with four types of siRNA sequences not targeting human genomes (Table 1).

**[Table 1]**

| Vector name | Target gene | Seq |
|---|---|---|
| PRDM1/RCOR1 double knockdown vector | PRDM1_1 | ggatgaacatctacttcta (SEQ ID NO 1) |
| | PRDM1_2 | gaatcaatgaagaaatcga (SEQ ID NO 2) |
| | RCOR1_1 | ccagataaatctatagcaa (SEQ ID NO 3) |
| | RCOR1_2 | gaacatggtaaagaagaga (SEQ ID NO 4) |
| Control knockdown vector | Control_1 | gtaatgcagaagaagacta (SEQ ID NO 5) |
| | Control_2 | gctccaaggtgtacgtgaa (SEQ ID NO 6) |
| | Control_3 | gctgcttcatctacaaggt (SEQ ID NO 7) |
| | Control_4 | gacgtcatcaaggagttca (SEQ ID NO 8) |

The CAR-T cells were cultured by using a plate coated with a mesothelin protein (MSN-H526x, ACROBiosystems AG) to apply antigen stimuli to the CAR-T cells. The CAR-T cells before the stimulation and the CAR-T cells after three times of the stimulation were stained with Brilliant Violet 421 anti-CCR7 (Cat. No.: 353208 manufactured by BioLegend, Inc.) and APC/cy7 anti-CD62L (Cat. No.: 304814 manufactured by BioLegend, Inc.) and then were subjected to flow cytometer analysis. As shown in Fig. 29, the frequency of the CD62L+CCR7+ cells increased owing to PRDM1/RCOR1 double knockdown between before and after the antigen stimulation.

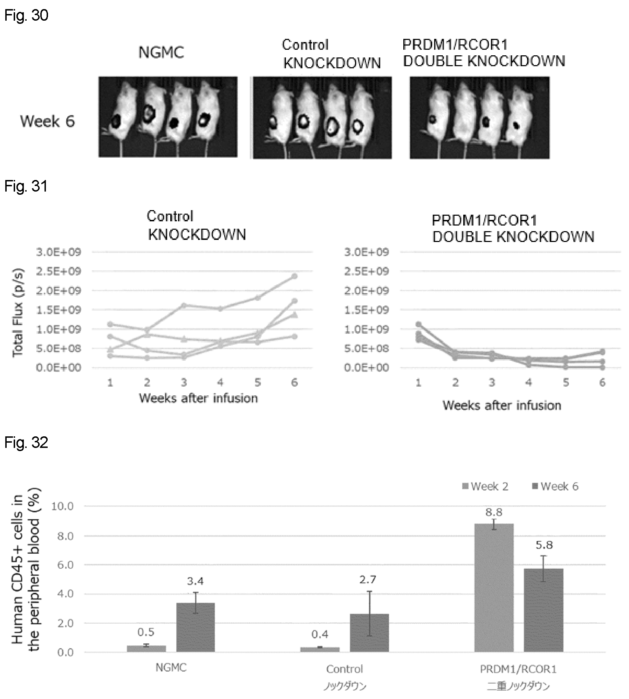

An in vivo therapeutic efficacy of the PRDM1/RCOR1 double knockdown CAR-T cells was examined. The PRDM1/RCOR1 double knockdown or control knockdown CAR-T cells or non-gene-modified cells (NGMC) were injected into each of NOG mice (In-Vivo Science Inc.) having AsPC-1/CMV-Luc (JCRB1454). In the group of mice into which the PRDM1/RCOR1 double knockdown CAR-T cells were injected, tumor growth was significantly repressed after six weeks (Fig. 30) and increase in total flux over time was suppressed (Fig. 31) as compared to the control knockout group. In this manner, the PRDM1/RCOR1 double knockdown CAR-T cells exhibited excellent therapeutic efficacy (Figs. 30 and 31) and exhibited prominent persistence in peripheral blood as well (Fig. 32).

### [Sequence Listing]

P24-176WO_PCT_long-lived T cells, pharmaceutical_20241020_212508_1.xml

## Claims

1. AT cell that expresses a recombinant receptor, the T cell that satisfies the following (I) and (II):
(I) the T cell has reduced or eliminated expression and/or function of the PRDM1 gene; and
(II) the T cell has reduced or eliminated expression and/or function of the RCOR1 gene and/or has increased or activated expression of the c-Jun gene.

2. The T cell according to claim 1, wherein the recombinant receptor is a chimeric antigen receptor (CAR).

3. The T cell according to claim 1, wherein the recombinant receptor is a chimeric antigen receptor (CAR) that specifically recognizes a tumor antigen.

4. The T cell according to claim 3, wherein the tumor antigen is CD19.

5. The T cell according to any one of claims 1 to 4, wherein the T cell is selected from the group consisting of a CD3+ T cell, a CD8+ T cell, a CD4+ T cell, an immune effector cell, a natural killer (NK) T cell, a γδ T cell, a combination of a CD4+ cell and a CD8T+ cell, a memory cell, a cytokine-induced killer cell, a tumor-infiltrating lymphocyte, and a combination thereof.

6. The T cell according to claim 1, wherein the cell further comprises: an inhibitor for the PRDM1 gene; and at least one of an inhibitor for the RCOR1 gene and an activator for the c-Jun gene.

7. The T cell according to claim 6, wherein
the inhibitor for the PRDM1 gene is the following (1) or (2):
(1) a gene editing system that targets one or more sites inside the PRDM1 gene or a regulatory element of the PRDM1 gene; or
(2) a nucleic acid that encodes one or more components of the gene editing system.

8. The T cell according to claim 6, wherein
the inhibitor for the RCOR1 gene is the following (1) or (2):
(1) a gene editing system that targets one or more sites inside the RCOR1 gene or a regulatory element of the RCOR1 gene; or
(2) a nucleic acid that encodes one or more components of the gene editing system.

9. The T cell according to claim 6, wherein the activator for the c-Jun gene is integration of the c-Jun gene into a cellular DNA via a c-Jun-expressing virus or vector.

10. A pharmaceutical composition for treating a disease in a subject, the pharmaceutical composition comprising the T cell according to claim 1.

11. The pharmaceutical composition according to claim 10, wherein the disease is a tumor or a cancer.

12. The pharmaceutical composition according to claim 10, wherein the T cell maintains an early memory phenotype and performs enhanced or maintained cytokine production, has reduced expression of an exhaustion marker, and/or exhibits suppressed reduction in an effector function as compared to a control T cell that satisfies neither the (I) nor the (II).

13. A method for enhancing a therapeutic efficacy of a T cell that expresses a recombinant receptor that specifically recognizes a tumor antigen, the method comprising:
reducing or eliminating expression and/or function of the PRDM1 gene in the T cell; and
reducing or eliminating expression and/or function of the RCOR1 gene in the T cell, and/or increasing or activating expression of the c-Jun gene in the T cell.

14. A method for enhancing a therapeutic efficacy of a T cell that expresses a recombinant receptor that specifically recognizes a tumor antigen, the method comprising:
bringing the T cell into contact with an inhibitor for the PRDM1 gene; and
bringing the T cell into contact with an inhibitor for the RCOR1 gene and/or bringing the T cell into contact with an activator for the c-Jun gene.

15. Use of an inhibitor for the PRDM1 gene and an inhibitor for the RCOR1 gene and/or an activator for the c-Jun gene in manufacturing of a pharmaceutical for a CAR-expressing cell therapy.
